# EUROPEAN PATENT APPLICATION

(11) **EP 2 184 609 A2**
(43) Date of publication of application: **12.05.2010**
(21) Application number: 10151397.6
(22) Date of filing: 18.03.2005
(51) Int. Cl.: G01N 33/574, C12N 15/11

(54) **Compositions and methods for treating lung cancer**

(30) Priority: 24.03.2004 US 555757 P
(62) Divisional of application: 05721548.5
(71) Applicant: Oncotherapy Science, Inc., Kawasaki-shi Kanagawa 213-0012 (JP)
(72) Inventor: Nakamura, Yusuke, Tokyo 113-8654 (JP); Daigo, Yataro, Tokyo 113-8654 (JP); Nakatsuru, Shuichi, Kawasaki-shi Kanagawa 213-0012 (JP)
(74) Representative: Vossius & Partner

(57) **Abstract**

The invention features a method for inhibiting growth of a cancer cell by contacting the cell with a composition of a PKP3, CDCA1, CDCA8, DLX5, URLC11 or NPTX1 siRNA. Methods of treating cancer are also within the invention. The invention also features products, including nucleic acid sequences and vectors as well as to compositions comprising them, useful in the provided methods. The invention also provides a method for inhibiting of tumor cell, for example lung cancer cell, particularly NSCLC.

SEQUENCE LISTING

<110>  Oncotherapy Science, Inc.

<120>  COMPOSITIONS AND METHODS FOR TREATING LUNG CANCER

<130>  M2811 EP/1 S3

<150>  US 60/555,757
<151>  2004-03-24

<160>  103

<170>  PatentIn version 3.1

<210>  1
<211>  2831
<212>  DNA
<213>  Homo sapiens

<400>  1
gaattccgga caggacgtga agatagttgg gtttggaggc ggccgccagg cccaggcccg      60

gtggacctgc cgccatgcag gacggtaact tcctgctgtc ggccctgcag cctgaggccg     120

gcgtgtgctc cctggcgctg ccctctgacc tgcagctgga ccgccggggc gccgaggggc     180

cggaggccga gcggctgcgg gcagcccgcg tccaggagca ggtccgcgcc cgcctcttgc     240

agctgggaca gcagccgcgg cacaacgggg ccgctgagcc cgagcctgag gccgagactg     300

ccagaggcac atccaggggg cagtaccaca ccctgcaggc tggcttcagc tctcgctctc     360

agggcctgag tggggacaag acctcgggct tccggcccat cgccaagccg gcctacagcc     420

cagcctcctg gtcctcccgc tccgccgtgg atctgagctg cagtcggagg ctgagttcag     480

cccacaatgg gggcagcgcc tttggggccg ctgggtacgg gggtgcccag cccacccctc     540

ccatgcccac caggcccgtg tccttccatg agcgcggtgg ggttgggagc cgggccgact     600

atgacacact ctccctgcgc tcgctgcggc tggggcccgg gggcctggac gaccgctaca     660

gcctggtgtc tgagcagctg gagcccgcgg ccacctccac ctacagggcc tttgcgtacg     720

agcgccaggc cagctccagc tccagccggg caggggggct ggactggccc gaggccactg     780

aggtttcccc gagccggacc atccgtgccc ctgccgtgcg gaccctgcag cgattccaga     840

gcagccaccg gagccgcggg gtaggcgggg cagtgccggg ggccgtcctg gagccagtgg     900

ctcgagcgcc atctgtgcgc agcctcagcc tcagcctggc tgactcgggc cacctgccgg 960

acgtgcatgg gttcaacagc tacggtagcc accgaaccct gcagagactc agcagcggtt 1020

ttgatgacat tgacctgccc tcagcagtca agtacctcat ggcttcagac cccaacctgc 1080

aggtgctggg agcggcctac atccagcaca agtgctacag cgatgcagcc gccaagaagc 1140

aggcccgcag ccttcaggcc gtgcctaggc tggtgaagct cttcaaccac gccaaccagg 1200

aagtgcagcg ccatgccaca ggtgccatgc gcaacctcat ctacgacaac gctgacaaca 1260

agctggccct ggtggaggag aacgggatct tcgagctgct gcggacactg cgggagcagg 1320

atgatgagct tcgcaaaaat gtcacaggga tcctgtggaa cctttcatcc agcgaccacc 1380

tgaaggaccg cctggccaga gacacgctgg agcagctcac ggacctggtg ttgagccccc 1440

tgtcgggggc tgggggtccc cccctcatcc agcagaacgc ctcggaggcg gagatcttct 1500

acaacgccac cggcttcctc aggaacctca gctcagcctc tcaggccact cgccagaaga 1560

tgcgggagtg ccacgggctg gtggacgccc tggtcacctc tatcaaccac gccctggacg 1620

cgggcaaatg cgaggacaag agcgtggaga acgcggtgtg cgtcctgcgg aacctgtcct 1680

accgcctcta cgacgagatg ccgccgtccg cgctgcagcg gctggagggt cgcggccgca 1740

gggacctggc gggggcgccg ccgggagagg tcgtgggctg cttcacgccg cagagccggc 1800

ggctgcgcga gctgcccctc gccgccgatg cgctcacctt cgcggaggtg tccaaggacc 1860

ccaagggcct cgagtggctg tggagccccc agatcgtggg gctgtacaac cggctgctgc 1920

agcgctgcga gctcaaccgg cacacgacgg aggcggccgc cggggcgctg cagaacatca 1980

cggcaggcga ccgcaggtgg gcgggggtgc tgagccgcct ggccctggag caggagcgta 2040

ttctgaaccc cctgctagac cgtgtcagga ccgccgacca ccaccagctg cgctcactga 2100

ctggcctcat ccgaaacctg tctcggaacg ctaggaacaa ggacgagatg tccacgaagg 2160

tggtgagcca cctgatcgag aagctgccag gcagcgtggg tgagaagtcg cccccagccg 2220

aggtgctggt caacatcata gctgtgctca acaacctggt ggtggccagc cccatcgctg 2280

cccgagacct gctgtatttt gacggactcc gaaagctcat cttcatcaag aagaagcggg 2340

acagccccga cagtgagaag tcctcccggg cagcatccag cctcctggcc aacctgtggc 2400

agtacaacaa gctccaccgt gactttcggg cgaagggcta tcggaaggag gacttcctgg 2460

gcccataggt gaagccttct ggaggagaag gtgacgtggc ccagcgtcca agggacagac 2520

tcagctccag gctgcttggc agcccagcct ggaggagaag gctaatgacg gaggggcccc 2580

tcgctggggc ccctgtgtgc atctttgagg gtcctgggcc accaggaggg gcagggtctt 2640

atagctgggg acttggcttc cgcagggcag ggggtggggc agggctcaag gctgctctgg 2700

tgtatggggt ggtgacccag tcacattggc agaggtgggg gttggctgtg gcctggcagt 2760

atcttgggat agccagcact gggaataaag atggccatga acagtcacaa aaaaaaaaaa 2820

aaaaggaatt c 2831

<210> 2
<211> 797
<212> PRT
<213> Homo sapiens

<400> 2

Met Gln Asp Gly Asn Phe Leu Leu Ser Ala Leu Gln Pro Glu Ala Gly
1               5                   10                  15

Val Cys Ser Leu Ala Leu Pro Ser Asp Leu Gln Leu Asp Arg Arg Gly
            20                  25                  30

Ala Glu Gly Pro Glu Ala Glu Arg Leu Arg Ala Ala Arg Val Gln Glu
        35                  40                  45

Gln Val Arg Ala Arg Leu Leu Gln Leu Gly Gln Gln Pro Arg His Asn
    50                  55                  60

Gly Ala Ala Glu Pro Glu Pro Glu Ala Glu Thr Ala Arg Gly Thr Ser
65                  70                  75                  80

Arg Gly Gln Tyr His Thr Leu Gln Ala Gly Phe Ser Ser Arg Ser Gln
                85                  90                  95

Gly Leu Ser Gly Asp Lys Thr Ser Gly Phe Arg Pro Ile Ala Lys Pro
            100                 105                 110

Ala Tyr Ser Pro Ala Ser Trp Ser Ser Arg Ser Ala Val Asp Leu Ser
        115                 120                 125

Cys Ser Arg Arg Leu Ser Ser Ala His Asn Gly Gly Ser Ala Phe Gly
    130                 135                 140

Ala Ala Gly Tyr Gly Gly Ala Gln Pro Thr Pro Pro Met Pro Thr Arg
145                 150                 155                 160

Pro Val Ser Phe His Glu Arg Gly Gly Val Gly Ser Arg Ala Asp Tyr
                165                 170                 175

Asp Thr Leu Ser Leu Arg Ser Leu Arg Leu Gly Pro Gly Gly Leu Asp
            180                 185                 190

Asp Arg Tyr Ser Leu Val Ser Glu Gln Leu Glu Pro Ala Ala Thr Ser
        195                 200                 205

Thr Tyr Arg Ala Phe Ala Tyr Glu Arg Gln Ala Ser Ser Ser Ser Ser
    210                 215                 220

Arg Ala Gly Gly Leu Asp Trp Pro Glu Ala Thr Glu Val Ser Pro Ser
225                 230                 235                 240

Arg Thr Ile Arg Ala Pro Ala Val Arg Thr Leu Gln Arg Phe Gln Ser
                245                 250                 255

Ser His Arg Ser Arg Gly Val Gly Gly Ala Val Pro Gly Ala Val Leu
            260                 265                 270

Glu Pro Val Ala Arg Ala Pro Ser Val Arg Ser Leu Ser Leu Ser Leu
        275                 280                 285

Ala Asp Ser Gly His Leu Pro Asp Val His Gly Phe Asn Ser Tyr Gly
    290                 295                 300

Ser His Arg Thr Leu Gln Arg Leu Ser Ser Gly Phe Asp Asp Ile Asp
305                 310                 315                 320

Leu Pro Ser Ala Val Lys Tyr Leu Met Ala Ser Asp Pro Asn Leu Gln
                325                 330                 335

Val Leu Gly Ala Ala Tyr Ile Gln His Lys Cys Tyr Ser Asp Ala Ala
            340                 345                 350

Ala Lys Lys Gln Ala Arg Ser Leu Gln Ala Val Pro Arg Leu Val Lys
        355                 360                 365

Leu Phe Asn His Ala Asn Gln Glu Val Gln Arg His Ala Thr Gly Ala
    370                 375                 380

Met Arg Asn Leu Ile Tyr Asp Asn Ala Asp Asn Lys Leu Ala Leu Val
385                 390                 395                 400

Glu Glu Asn Gly Ile Phe Glu Leu Leu Arg Thr Leu Arg Glu Gln Asp
                405                 410                 415

Asp Glu Leu Arg Lys Asn Val Thr Gly Ile Leu Trp Asn Leu Ser Ser
            420                 425                 430

Ser Asp His Leu Lys Asp Arg Leu Ala Arg Asp Thr Leu Glu Gln Leu
        435                 440                 445

Thr Asp Leu Val Leu Ser Pro Leu Ser Gly Ala Gly Gly Pro Pro Leu
    450                 455                 460

Ile Gln Gln Asn Ala Ser Glu Ala Glu Ile Phe Tyr Asn Ala Thr Gly
465                 470                 475                 480

Phe Leu Arg Asn Leu Ser Ser Ala Ser Gln Ala Thr Arg Gln Lys Met
                485                 490                 495

Arg Glu Cys His Gly Leu Val Asp Ala Leu Val Thr Ser Ile Asn His
            500                 505                 510

Ala Leu Asp Ala Gly Lys Cys Glu Asp Lys Ser Val Glu Asn Ala Val

        515                 520                 525

Cys Val Leu Arg Asn Leu Ser Tyr Arg Leu Tyr Asp Glu Met Pro Pro
    530                 535                 540

Ser Ala Leu Gln Arg Leu Glu Gly Arg Gly Arg Arg Asp Leu Ala Gly
545                 550                 555                 560

Ala Pro Pro Gly Glu Val Val Gly Cys Phe Thr Pro Gln Ser Arg Arg
                565                 570                 575

Leu Arg Glu Leu Pro Leu Ala Ala Asp Ala Leu Thr Phe Ala Glu Val
            580                 585                 590

Ser Lys Asp Pro Lys Gly Leu Glu Trp Leu Trp Ser Pro Gln Ile Val
        595                 600                 605

Gly Leu Tyr Asn Arg Leu Leu Gln Arg Cys Glu Leu Asn Arg His Thr
    610                 615                 620

Thr Glu Ala Ala Ala Gly Ala Leu Gln Asn Ile Thr Ala Gly Asp Arg
625                 630                 635                 640

Arg Trp Ala Gly Val Leu Ser Arg Leu Ala Leu Glu Gln Glu Arg Ile
                645                 650                 655

Leu Asn Pro Leu Leu Asp Arg Val Arg Thr Ala Asp His His Gln Leu
            660                 665                 670

Arg Ser Leu Thr Gly Leu Ile Arg Asn Leu Ser Arg Asn Ala Arg Asn
        675                 680                 685

Lys Asp Glu Met Ser Thr Lys Val Val Ser His Leu Ile Glu Lys Leu
    690                 695                 700

Pro Gly Ser Val Gly Glu Lys Ser Pro Pro Ala Glu Val Leu Val Asn
705                 710                 715                 720

Ile Ile Ala Val Leu Asn Asn Leu Val Val Ala Ser Pro Ile Ala Ala
                725                 730                 735

Arg Asp Leu Leu Tyr Phe Asp Gly Leu Arg Lys Leu Ile Phe Ile Lys
            740                 745                 750

Lys Lys Arg Asp Ser Pro Asp Ser Glu Lys Ser Ser Arg Ala Ala Ser
        755                 760                 765

Ser Leu Leu Ala Asn Leu Trp Gln Tyr Asn Lys Leu His Arg Asp Phe
    770                 775                 780

Arg Ala Lys Gly Tyr Arg Lys Glu Asp Phe Leu Gly Pro
785                 790                 795

<210>  3
<211>  2003
<212>  DNA
<213>  Homo sapiens

<400>  3
ggaatggggc gggacttcca gtaggaggcg gcaagtttga aaagtgatga cggttgacgt      60

ttgctgattt ttgactttgc ttgtagctgc tccccgaact cgccgtcttc ctgtcggcgg     120

ccggcactgt aggtgagcgc gagaggacgg aggaaggaag cctgcagaca gacgccttct     180

ccatcccaag gcgcgggcag gtgccgggac gctgggcctg gcggtgtttt cgtcgtgctc     240

agcggtggga ggaggcggaa gaaaccagag cctgggagat taacaggaaa cttccaagat     300

ggaaactttg tctttcccca gatataatgt agctgagatt gtgattcata ttcgcaataa     360

gatcttaaca ggagctgatg gtaaaaacct caccaagaat gatctttatc caaatccaaa     420

gcctgaagtc ttgcacatga tctacatgag agccttacaa atagtctatg gaattcgact     480

ggaacatttt tacatgatgc cagtgaactc tgaagtcatg tatccacatt taatggaagg     540

cttcttacca ttcagcaatt tagttactca tctggactca tttttgccta tctgccgggt     600

gaatgacttt gagactgctg atattctatg tccaaaagca aaacggacaa gtcggttttt     660

aagtggcatt atcaacttta ttcacttcag agaagcatgc cgtgaaacgt atatggaatt     720

tctttggcaa tataaatcct ctgcggacaa aatgcaacag ttaaacgccg cacaccagga     780

ggcattaatg aaactggaga gacttgattc tgttccagtt gaagagcaag aagagttcaa 840

gcagctttca gatggaattc aggagctaca acaatcacta aatcaggatt ttcatcaaaa 900

aacgatagtg ctgcaagagg gaaattccca aaagaagtca aatatttcag agaaaaccaa 960

gcgtttgaat gaactaaaat tgttggtggt ttctttgaaa gaaatacaag agagtttgaa 1020

aacaaaaatt gtggattctc cagagaagtt aaagaattat aaagaaaaaa tgaaagatac 1080

ggtccagaag cttaaaaatg ccagacaaga agtggtggag aaatatgaaa tctatggaga 1140

ctcagttgac tgcctgcctt catgtcagtt ggaagtgcag ttatatcaaa agaaaataca 1200

ggacctttca gataataggg aaaaattagc cagtatctta aaggagagcc tgaacttgga 1260

ggaccaaatt gagagtgatg agtcagaact gaagaaattg aagactgaag aaaattcgtt 1320

caaaagactg atgattgtga agaaggaaaa acttgccaca gcacaattca aaataaataa 1380

gaagcatgaa gatgttaagc aatacaaacg cacagtaatt gaggattgca ataaagttca 1440

agaaaaaaga ggtgctgtct atgaacgagt aaccacaatt aatcaagaaa tccaaaaaat 1500

taaacttgga attcaacaac taaaagatgc tgctgaaagg gagaaactga agtcccagga 1560

aatatttcta aacttgaaaa ctgctttgga gaaataccac gacggtattg aaaaggcagc 1620

agaggactcc tatgctaaga tagatgagaa gacagctgaa ctgaagagga agatgttcaa 1680

aatgtcaacc tgattaacaa aattacatgt ctttttgtaa atggcttgcc atcttttaat 1740

tttctattta gaaagaaaag ttgaagcgaa tggaagtatc agaagtacca aataatgttg 1800

gcttcatcag tttttataca ctctcataag tagttaataa gatgaattta atgtaggctt 1860

ttattaattt ataattaaaa taacttgtgc agctattcat gtctctactc tgccccttgt 1920

tgtaaatagt ttgagtaaaa caaaactagt tacctttgaa atatatatat ttttttctgt 1980

taaaaaaaaa aaaaaaaaaa aaa 2003

<210> 4
<211> 464
<212> PRT
<213> Homo sapiens

<400> 4

Met Glu Thr Leu Ser Phe Pro Arg Tyr Asn Val Ala Glu Ile Val Ile
1               5                   10                  15

His Ile Arg Asn Lys Ile Leu Thr Gly Ala Asp Gly Lys Asn Leu Thr
            20                  25                  30

Lys Asn Asp Leu Tyr Pro Asn Pro Lys Pro Glu Val Leu His Met Ile
        35                  40                  45

Tyr Met Arg Ala Leu Gln Ile Val Tyr Gly Ile Arg Leu Glu His Phe
    50                  55                  60

Tyr Met Met Pro Val Asn Ser Glu Val Met Tyr Pro His Leu Met Glu
65                  70                  75                  80

Gly Phe Leu Pro Phe Ser Asn Leu Val Thr His Leu Asp Ser Phe Leu
                85                  90                  95

Pro Ile Cys Arg Val Asn Asp Phe Glu Thr Ala Asp Ile Leu Cys Pro
            100                 105                 110

Lys Ala Lys Arg Thr Ser Arg Phe Leu Ser Gly Ile Ile Asn Phe Ile
        115                 120                 125

His Phe Arg Glu Ala Cys Arg Glu Thr Tyr Met Glu Phe Leu Trp Gln
    130                 135                 140

Tyr Lys Ser Ser Ala Asp Lys Met Gln Gln Leu Asn Ala Ala His Gln
145                 150                 155                 160

Glu Ala Leu Met Lys Leu Glu Arg Leu Asp Ser Val Pro Val Glu Glu
                165                 170                 175

Gln Glu Glu Phe Lys Gln Leu Ser Asp Gly Ile Gln Glu Leu Gln Gln
            180                 185                 190

Ser Leu Asn Gln Asp Phe His Gln Lys Thr Ile Val Leu Gln Glu Gly
        195                 200                 205

Asn Ser Gln Lys Lys Ser Asn Ile Ser Glu Lys Thr Lys Arg Leu Asn
    210                 215                 220

Glu Leu Lys Leu Leu Val Val Ser Leu Lys Glu Ile Gln Glu Ser Leu
225                 230                 235                 240

Lys Thr Lys Ile Val Asp Ser Pro Glu Lys Leu Lys Asn Tyr Lys Glu
                245                 250                 255

Lys Met Lys Asp Thr Val Gln Lys Leu Lys Asn Ala Arg Gln Glu Val
            260                 265                 270

Val Glu Lys Tyr Glu Ile Tyr Gly Asp Ser Val Asp Cys Leu Pro Ser
        275                 280                 285

Cys Gln Leu Glu Val Gln Leu Tyr Gln Lys Lys Ile Gln Asp Leu Ser
    290                 295                 300

Asp Asn Arg Glu Lys Leu Ala Ser Ile Leu Lys Glu Ser Leu Asn Leu
305                 310                 315                 320

Glu Asp Gln Ile Glu Ser Asp Glu Ser Glu Leu Lys Lys Leu Lys Thr
                325                 330                 335

Glu Glu Asn Ser Phe Lys Arg Leu Met Ile Val Lys Lys Glu Lys Leu
            340                 345                 350

Ala Thr Ala Gln Phe Lys Ile Asn Lys Lys His Glu Asp Val Lys Gln
        355                 360                 365

Tyr Lys Arg Thr Val Ile Glu Asp Cys Asn Lys Val Gln Glu Lys Arg
    370                 375                 380

Gly Ala Val Tyr Glu Arg Val Thr Thr Ile Asn Gln Glu Ile Gln Lys
385                 390                 395                 400

Ile Lys Leu Gly Ile Gln Gln Leu Lys Asp Ala Ala Glu Arg Glu Lys
                405                 410                 415

Leu Lys Ser Gln Glu Ile Phe Leu Asn Leu Lys Thr Ala Leu Glu Lys
            420                 425                 430

Tyr His Asp Gly Ile Glu Lys Ala Ala Glu Asp Ser Tyr Ala Lys Ile
        435                 440                 445

Asp Glu Lys Thr Ala Glu Leu Lys Arg Lys Met Phe Lys Met Ser Thr
    450                 455                 460

<210>  5
<211>  2307
<212>  DNA
<213>  Homo sapiens

<400>  5
gtgactgtgg agtttgaatt gggtggcggt tgactgtaga gccgctctct ctcactggca      60

cagcgaggtt ttgctcagcc cttgtctcgg gaccgcagcc tccgccgagc gccatggctc     120

ctaggaaggg cagtagtcgg gtggccaaga ccaactcctt acggaggcgg aagctcgcct     180

cctttctgaa agacttcgac cgtgaagtgg aaatacgaat caagcaaatt gagtcagaca     240

ggcagaacct cctcaaggag gtggataacc tctacaacat cgagatcctg cggctcccca     300

aggctctgcg cgagatgaac tggcttgact acttcgccct tggaggaaac aaacaggccc     360

tggaagaggc ggcaacagct gacctggata tcaccgaaat aaacaaacta acagcagaag     420

ctattcagac acccctgaaa tctgccaaaa cacgaaaggt aatacaggta gatgaaatga     480

tagtggaaga ggaagaagaa gaagaaaatg aacgtaagaa tcttcaaact gcaagagtca     540

aaaggtgtcc tccatccaag aagagaactc agtccataca aggaaaagga aaagggaaaa     600

ggtcaagccg tgctaacact gttaccccag ccgtgggccg attggaggtg tccatggtca     660

aaccaactcc aggcctgaca cccaggtttg actcaagggt cttcaagacc cctggcctgc     720

gtactccagc agcaggagag cggatttaca acatctcagg gaatggcagc cctcttgctg     780

acagcaaaga gatcttcctc actgtgccag tgggcggcgg agagagcctg cgattattgg     840

ccagtgactt gcagaggcac agtattgccc agctggatcc agaggccttg ggaaacatta     900

agaagctctc caaccgtctc gcccaaatct gcagcagcat acggacccac aaatgagaca     960

ccaaagttga caggatggac ttttaatggg cacttctggg accctgaaga gacttcttcc 1020

cttcaggctt attgtttgag tgtgaagttc cagagcaagg agccatgttc ctctaaggga 1080

attcaggaat tcagacgtgc tagtcccaca ccagttaggt agagctgtct gttcaccctc 1140

ccatcccagc tgatcccagt cactgcttgc tggggccatg ccatggaagc ttcccatcag 1200

tctcccagct gaatcctccc tgctctctga gctgctgcct tttgcctcct gcaactcaac 1260

atcctcttca ccctgccctg cctgcagttg agggggcgaa gaagaaccct gtgttctcag 1320

gaagactgcc tccaccaccg ctacccagag aacctctgca tctggcattt ctgctctcta 1380

tgcttgagac cgggaggttt aggctcagat aagtgagctc tgggccatga gagggtaggt 1440

ccagaaggtg gggggaactg tacagatcag cagagcagga cagttggcag cagtgacctc 1500

agtagggaac atgtccgtct accctctcgc actcatgaca cctcccccta ccagccctcc 1560

tcttcctcct cctcctcctc ctgtgggagg tggtcagtgg gacttaggga tctttcacct 1620

gctgtgccca gtagttctga agtctgcttg tggagcagtg ttttatgttt atccctgttt 1680

actgaagacc aaatactggt ttggagacaa cttccatgtc ttgctcttct acctccctag 1740

ttagtggaaa tttggataag ggaactgtag ggcccagatt ctggaggttt tatgtcattg 1800

gccacagaat aactgtctct aagctatcca tggtccagtg gtccctgcca agtctgtaga 1860

cttcagagag cacttctctc ttatggggtt catggaaaca ggggcgggtg tgacttgctt 1920

ggtggcctca ttccatgtgt gcctgtgcct ggggcatgga ctttgttaag cagagtcagc 1980

agtgaggtcc tcattctcca gccagcctct ctgccctgga gaatcatgtg ctatgttcta 2040

agaatttgag aactagagtc ctcatcccca ggcttgaagg cacatggctt tctcatgtag 2100

ggctctctgt ggtatttgtt attattttgc aacaagacca ttttagtaaa acagtcctgt 2160

tcaagttgta ttcttttaag ttcttttatt ctcctttccc tgagattttt gtatatattg 2220

ttctgagtaa tggtatcttt gagctgattg ttctaatcag agctggtacc tactttcaat 2280

aaattctggt tttgtgtttt cttctgt 2307

<210> 6
<211> 280
<212> PRT

<213>  Homo sapiens

<400>  6

Met Ala Pro Arg Lys Gly Ser Ser Arg Val Ala Lys Thr Asn Ser Leu
1               5                   10                  15

Arg Arg Arg Lys Leu Ala Ser Phe Leu Lys Asp Phe Asp Arg Glu Val
            20                  25                  30

Glu Ile Arg Ile Lys Gln Ile Glu Ser Asp Arg Gln Asn Leu Leu Lys
        35                  40                  45

Glu Val Asp Asn Leu Tyr Asn Ile Glu Ile Leu Arg Leu Pro Lys Ala
    50                  55                  60

Leu Arg Glu Met Asn Trp Leu Asp Tyr Phe Ala Leu Gly Gly Asn Lys
65                  70                  75                  80

Gln Ala Leu Glu Glu Ala Ala Thr Ala Asp Leu Asp Ile Thr Glu Ile
                85                  90                  95

Asn Lys Leu Thr Ala Glu Ala Ile Gln Thr Pro Leu Lys Ser Ala Lys
            100                 105                 110

Thr Arg Lys Val Ile Gln Val Asp Glu Met Ile Val Glu Glu Glu Glu
        115                 120                 125

Glu Glu Glu Asn Glu Arg Lys Asn Leu Gln Thr Ala Arg Val Lys Arg
    130                 135                 140

Cys Pro Pro Ser Lys Lys Arg Thr Gln Ser Ile Gln Gly Lys Gly Lys
145                 150                 155                 160

Gly Lys Arg Ser Ser Arg Ala Asn Thr Val Thr Pro Ala Val Gly Arg
                165                 170                 175

Leu Glu Val Ser Met Val Lys Pro Thr Pro Gly Leu Thr Pro Arg Phe
            180                 185                 190

Asp Ser Arg Val Phe Lys Thr Pro Gly Leu Arg Thr Pro Ala Ala Gly
        195                 200                 205

Glu Arg Ile Tyr Asn Ile Ser Gly Asn Gly Ser Pro Leu Ala Asp Ser
    210                 215                 220

Lys Glu Ile Phe Leu Thr Val Pro Val Gly Gly Gly Glu Ser Leu Arg
225                 230                 235                 240

Leu Leu Ala Ser Asp Leu Gln Arg His Ser Ile Ala Gln Leu Asp Pro
                245                 250                 255

Glu Ala Leu Gly Asn Ile Lys Lys Leu Ser Asn Arg Leu Ala Gln Ile
            260                 265                 270

Cys Ser Ser Ile Arg Thr His Lys
        275                 280

<210>  7
<211>  1419
<212>  DNA
<213>  Homo sapiens

<400>  7
cggagacaga gacttcacga ctcccagtct cctcctcgcc gcggccgccg cctcctcctt      60

ctctcctcct cctcttcctc ctcctccctc gctcccacag ccatgtctgc ttagaccaga     120

gcagccccac agccaactag ggcagctgcc gccgccacaa cagcaaggac agccgctgcc     180

gccgcccgtg agcgatgaca ggagtgtttg acagaagggt ccccagcatc cgatccggcg     240

acttccaagc tccgttccag acgtccgcag ctatgcacca tccgtctcag gaatcgccaa     300

ctttgcccga gtcttcagct accgattctg actactacag ccctacgggg ggagccccgc     360

acggctactg ctctcctacc tcggcttcct atggcaaagc tctcaacccc taccagtatc     420

agtatcacgg cgtgaacggc tccgccggga gctacccagc caaagcttat gccgactata     480

gctacgctag ctcctaccac cagtacggcg gcgcctacaa ccgcgtccca agcgccacca     540

accagccaga gaaagaagtg accgagcccg aggtgagaat ggtgaatggc aaaccaaaga     600

aagttcgtaa acccaggact atttattcca gctttcagct ggccgcatta cagagaaggt 660

ttcagaagac tcagtacctc gccttgccgg aacgcgccga gctggccgcc tcgctgggat 720

tgacacaaac acaggtgaaa atctggtttc agaacaaaag atccaagatc aagaagatca 780

tgaaaaacgg ggagatgccc ccggagcaca gtcccagctc cagcgaccca atggcgtgta 840

actcgccgca gtctccagcg gtgtgggagc cccagggctc gtcccgctcg ctcagccacc 900

accctcatgc ccaccctccg acctccaacc agtccccagc gtccagctac ctggagaact 960

ctgcatcctg gtacacaagt gcagccagct caatcaattc ccacctgccg ccgccgggct 1020

ccttacagca cccgctggcg ctggcctccg ggacactcta ttagatgggc tgctctctct 1080

tactctcttt tttgggacta ctgtgttttg ctgttctaga aaatcataaa gaaaggaatt 1140

catatgggga agttcggaaa actgaaaaag attcatgtgt aaagcttttt tttgcatgta 1200

agttattgca tttcaaaaga cccccccttt ttttacagag gacttttttt gcgcaactgt 1260

ggacactttc aatggtgcct tgaaatctat gacctcaact tttcaaaaga cttttttcaa 1320

tgttatttta gccatgtaaa taagtgtaga tagaggaatt aaactgtata ttctggataa 1380

ataaaattat ttcgaccatg aaaaaaaaaa aaaaaaaaa 1419

<210> 8
<211> 289
<212> PRT
<213> Homo sapiens

<400> 8

<210> 9
<211> 2539
<212> DNA
<213> Homo sapiens

<400> 9
atccttatct tctcgtctag ttctccgagg ttgaaggctc ggcctgctca gagaaggaaa      60

ctgaggtcca ccgagttgga gaaacctact caacaccagg actaacttct tcagtgctta     120

gagtgtgaga aaaatggcaa atatgaatag tgattctagg catcttggca cctctgaggt     180

agatcatgaa agagatcctg gacctatgaa tatccagttt gagccatcgg atctaagatc     240

caaaaggcct ttctgtatag agcccacaaa catcgtgaat gtgaatcatg tcattcagag     300

ggttagtgac catgcctctg ccatgaacaa gagaattcat tactacagcc ggctcaccac     360

tcctgcagac aaggcactga ttgccccaga ccatgtagtt ccagctccag aagagtgcta     420

tgtgtatagt ccattgggct ctgcttataa acttcaaagt tacactgaag gatacggtaa     480

aaacaccagt ttagtaacca tttttatgat ttggaatacc atgatgggaa catctatact     540

aagcattcct tggggcataa aacaggctgg atttactact ggaatgtgtg tcatcatact     600

gatgggcctt ttaacacttt attgctgcta cagagtagtg aaatcacgga ctatgatgtt     660

ttcattggat accactacct gggaatatcc agatgtctgc agacattatt tcggctcctt     720

tgggcagtgg tcgagtctcc tcttctcctt ggtgtctctc attggagcaa tgatagttta     780

ttgggtgctt atgtcaaatt ttctttttaa tactggaaag tttattttta attttattca     840

tcacattaat gacacagaca ctatactgag taccaataat agcaaccctg tgatttgtcc     900

aagtgccggg agtggaggcc atcctgacaa cagctctatg attttctatg ccaatgacac     960

aggagcccaa cagtttgaaa agtggtggga taagtccagg acagtcccct tttatcttgt    1020

agggctcctc ctcccactgc tcaatttcaa gtctccttca tttttttcaa aatttaatat    1080

cctaggcaca gtgtctgtcc tttatttgat tttccttgtc acctttaagg ctgttcgctt 1140
gggatttcat ttggaatttc attggtttat accaacagaa ttttttgtac cagagataag 1200
atttcagttt ccacagctga ctggagtgct tacccttgct ttttttattc ataattgtat 1260
catcacactc ttgaagaaca acaagaaaca agaaaacaat gtgagggact tgtgcattgc 1320
ttatatgctg gtgacattaa cttatctcta tattggagtc ctggtttttg cttcatttcc 1380
ttcaccacca ttatccaaag attgtattga gcagaatttt ttagacaact tccctagcag 1440
tgacaccctg tccttcattg caaggatatt cctgctgttc cagatgatga ctgtataccc 1500
actcttaggc tacctggctc gtgtccagct tttgggccat atcttcggtg acatttatcc 1560
tagcattttc catgtgctga ttcttaatct aattattgtg ggagctggag tgatcatggc 1620
ctgtttctac ccaaacatag gagggatcat aagatattca ggagcagcat gtggactggc 1680
ctttgtattc atatacccat ctctcatcta tataatttcc ctccaccaag aagagcgtct 1740
gacatggcct aaattaatct tccacgtttt catcatcatt ttgggcgtgg ctaacctgat 1800
tgttcagttt tttatgtgaa atacctcaac tgtttttttc aagagctctc atgatatttt 1860
gagccttgac aacagttcta tataaattca cttgtaaatg ctgctgttgt gtaattctaa 1920
atattttcta agataatttg aaagcaaggg aaatagtggc cccttaatga gtattttttt 1980
attggggtgg ggaaaggggc aaaaagaatg atcttagtgt ctttaccttt ctcatattaa 2040
ctcacctctt tattctgtgg tcttttctga atagaaatgt atgccctagg aagaaatcat 2100
gctgggtttt gcttttagag ataaaaggtg gtggatttat tttgcctgca gtaaagattc 2160
tcagggtgtc agagcagcat attgtcaaat cctgcttctg ttttatgttt cagtgtattc 2220
actttcattt tcttacttac tagaccattt ctgcagtttg cccaaacctc tactgtttgg 2280
gacagtaagc caaatacctc atttttaaaa agaagttttc atggcatcag tgttaataaa 2340
gtacattttt aactgagtct taatctctat ttgaagaaaa agtagagaca aaagtaatgt 2400
caatgtaatc cccaggatca tgaaatgtat acaaaataaa taaagtagga gagtttgttg 2460
ctgtctaact cctcttttat ttctaatcca gtaccagtga cttagatcga aattacagtt 2520
ccccaagcca gactttgca 2539

<210>  10
<211>  561
<212>  PRT
<213>  Homo sapiens

<400>  10

Met Ala Asn Met Asn Ser Asp Ser Arg His Leu Gly Thr Ser Glu Val
1               5                   10                  15

Asp His Glu Arg Asp Pro Gly Pro Met Asn Ile Gln Phe Glu Pro Ser
            20                  25                  30

Asp Leu Arg Ser Lys Arg Pro Phe Cys Ile Glu Pro Thr Asn Ile Val
        35                  40                  45

Asn Val Asn His Val Ile Gln Arg Val Ser Asp His Ala Ser Ala Met
    50                  55                  60

Asn Lys Arg Ile His Tyr Tyr Ser Arg Leu Thr Thr Pro Ala Asp Lys
65                  70                  75                  80

Ala Leu Ile Ala Pro Asp His Val Val Pro Ala Pro Glu Glu Cys Tyr
                85                  90                  95

Val Tyr Ser Pro Leu Gly Ser Ala Tyr Lys Leu Gln Ser Tyr Thr Glu
            100                 105                 110

Gly Tyr Gly Lys Asn Thr Ser Leu Val Thr Ile Phe Met Ile Trp Asn
        115                 120                 125

Thr Met Met Gly Thr Ser Ile Leu Ser Ile Pro Trp Gly Ile Lys Gln
    130                 135                 140

Ala Gly Phe Thr Thr Gly Met Cys Val Ile Ile Leu Met Gly Leu Leu
145                 150                 155                 160

Thr Leu Tyr Cys Cys Tyr Arg Val Val Lys Ser Arg Thr Met Met Phe
                165                 170                 175

Ser Leu Asp Thr Thr Thr Trp Glu Tyr Pro Asp Val Cys Arg His Tyr
            180                 185                 190

Phe Gly Ser Phe Gly Gln Trp Ser Ser Leu Leu Phe Ser Leu Val Ser
        195                 200                 205

Leu Ile Gly Ala Met Ile Val Tyr Trp Val Leu Met Ser Asn Phe Leu
    210                 215                 220

Phe Asn Thr Gly Lys Phe Ile Phe Asn Phe Ile His His Ile Asn Asp
225                 230                 235                 240

Thr Asp Thr Ile Leu Ser Thr Asn Asn Ser Asn Pro Val Ile Cys Pro
                245                 250                 255

Ser Ala Gly Ser Gly Gly His Pro Asp Asn Ser Ser Met Ile Phe Tyr
            260                 265                 270

Ala Asn Asp Thr Gly Ala Gln Gln Phe Glu Lys Trp Trp Asp Lys Ser
        275                 280                 285

Arg Thr Val Pro Phe Tyr Leu Val Gly Leu Leu Leu Pro Leu Leu Asn
    290                 295                 300

Phe Lys Ser Pro Ser Phe Phe Ser Lys Phe Asn Ile Leu Gly Thr Val
305                 310                 315                 320

Ser Val Leu Tyr Leu Ile Phe Leu Val Thr Phe Lys Ala Val Arg Leu
                325                 330                 335

Gly Phe His Leu Glu Phe His Trp Phe Ile Pro Thr Glu Phe Phe Val
            340                 345                 350

Pro Glu Ile Arg Phe Gln Phe Pro Gln Leu Thr Gly Val Leu Thr Leu
        355                 360                 365

Ala Phe Phe Ile His Asn Cys Ile Ile Thr Leu Leu Lys Asn Asn Lys
    370                 375                 380

Lys Gln Glu Asn Asn Val Arg Asp Leu Cys Ile Ala Tyr Met Leu Val
385                 390                 395                 400

Thr Leu Thr Tyr Leu Tyr Ile Gly Val Leu Val Phe Ala Ser Phe Pro
                405                 410                 415

Ser Pro Pro Leu Ser Lys Asp Cys Ile Glu Gln Asn Phe Leu Asp Asn
            420                 425                 430

Phe Pro Ser Ser Asp Thr Leu Ser Phe Ile Ala Arg Ile Phe Leu Leu
        435                 440                 445

Phe Gln Met Met Thr Val Tyr Pro Leu Leu Gly Tyr Leu Ala Arg Val
    450                 455                 460

Gln Leu Leu Gly His Ile Phe Gly Asp Ile Tyr Pro Ser Ile Phe His
465                 470                 475                 480

Val Leu Ile Leu Asn Leu Ile Ile Val Gly Ala Gly Val Ile Met Ala
                485                 490                 495

Cys Phe Tyr Pro Asn Ile Gly Gly Ile Ile Arg Tyr Ser Gly Ala Ala
            500                 505                 510

Cys Gly Leu Ala Phe Val Phe Ile Tyr Pro Ser Leu Ile Tyr Ile Ile
        515                 520                 525

Ser Leu His Gln Glu Glu Arg Leu Thr Trp Pro Lys Leu Ile Phe His
    530                 535                 540

Val Phe Ile Ile Ile Leu Gly Val Ala Asn Leu Ile Val Gln Phe Phe
545                 550                 555                 560

Met

<210>  11

<211>  5071
<212>  DNA
<213>  Homo sapiens

<400>  11
ggcctgatag cgcggcgtgt ggaccggccg aagagcgcgc ccagagcggc gccgtcgcga      60

gccacagccc gagccggtcc cagccgagcc gagccccagc cgagccgagc cggcccgagc     120

gctccggtgc ccgcagccat gccggccggc cgcgcgcgca cctgtgcgct gctcgccctc     180

tgcctcctgg ggccccagga tttcgggccg acgcgcttca tctgcacttc ggtgccggtg     240

gacgccgaca tgtgtgccgc gtccgtggcc gcgggcggcg cggaggagct ccggagcagc     300

aatgtgctgc agctccggga gaccgtgctg cagcagaagg agaccatcct gagccagaag     360

gagaccatcc gcgagctgac cgccaagctg ggccgctgcg agagccagag cacgctggac     420

cccggagccg gcgaggcccg ggcgggcggc ggccgcaagc agccgggctc gggcaagaac     480

accatgggcg acctgtcccg gacaccggcc gccgagacgc tcagccaact cgggcaaact     540

ttgcaatcgc tcaaaacccg cctggagaac ctcgagcagt acagccgcct caattcctcc     600

agccagacca acagcctcaa ggatctgctg cagagcaaga tcgatgagct ggagaggcag     660

gtgctgtccc gggtgaacac cctggaggag ggcaaggggg ggcccaagaa cgacaccgag     720

gagagggtca agatcgagac cgccctgacc tccctgcacc agcggatcag cgagctcgag     780

aaaggtcaga aagacaaccg ccctggagac aagttccagc tcacattccc actgcggacc     840

aactatatgt atgccaaggt gaagaagagc ctgccagaga tgtacgcctt cactgtctgc     900

atgtggctca agtccagcgc cacgccaggt gtgggcacgc ccttctccta cgctgtgccc     960

ggccaggcca acgagctggt cctcattgag tggggcaaca accccatgga gatcctcatc    1020

aatgacaagg tggccaagct gccttttgtc atcaatgatg gcaagtggca ccacatctgt    1080

gtcacctgga ccacccggga cggggtctgg gaggcctacc aggatggcac gcagggtggc    1140

agtggcgaga acttggcgcc ctatcacccc atcaagcccc agggcgtgct ggtgctgggc    1200

caggagcagg acactctggg tggtgggttt gatgccaccc aggcatttgt gggtgagctg    1260

gcccacttca acatctggga ccgcaagctg acccccgggg aggtgtacaa cctggccacc    1320

tgcagcacca aggctctgtc cggcaatgtc atcgcctggg ctgaatccca catcgagatc    1380

tacggcgggg ccaccaagtg gaccttcgag gcctgtcgcc agatcaactg agcacggcag 1440
gccaggctga gccgcccgcc ctcgccccct gcttgtgcgg cgatgatctg tttgtgcgtc 1500
tcttctctcc cttttcccca ggaatgaacc gaggccgtcg ccctgcacac gcacacgcac 1560
acagcctggt tttgtcctca tgcacacgaa gcagcccctg ctcccatctg tccctgagga 1620
agccccactt ctctgtagga gcccggactc tctcagcatg ccccattcac agctgaagtg 1680
ggtgctgcaa cgtcttgaac aaggcagaag ttggtgagag gatctgtgtg tgcgtgtcta 1740
catgtgtgtg tctacgtgtg tgcgtgcgtg gctgggggag gccttttctt tgaggacgta 1800
cctcatttcc ttctttcttc tggctttgga aaaatctcat gatgaaaatt catatttgcc 1860
aactttgtta gctgcgtgcg tgctttgggg ttggtgcaac ctcagtacac gcatttgtct 1920
ttgtttgcaa acctttctca gagcgacata tctttatatt gatgtaataa atgtctttta 1980
gtggtttgtc aaaggccggg ggcgggggct ctctacagag aatttttatt ttgtaataga 2040
agtgaactgt ctctgaaggt gaaggcaggc cgtcctggga tggtaccctg tgctctcccg 2100
tggaggagag gggatggctg aggacactgg cccttaccca gggcgaacag catccatccc 2160
tgctgtttgc atcttgagag cagcatgggg cctgggaggt cggcctgtgt gcccagctca 2220
gctagctctg ccccaggacg gccctgccct cgaccttccc acctcctcag atcctgcaag 2280
gctgggtctg cccctccctt ctcacctctg gagctgtgct gcactgcttc ccagagaggg 2340
ccctgagaga ggagcgtgcc acccaccagg gaagccgggc cccagcaccc ctctcctttg 2400
gcctcccgga gtgcagacca gaggggacct tttaaggaaa gaagccgtgt ttcgatgaag 2460
acctggccac atggggcact gggacttcaa cccagcccat cggtgggaag gtcctttttg 2520
ggggactttg acagccatat ccctcccagc acaccaggcg ccaggtgagc tggttcagac 2580
ccctccaggg tactccagag acctcacgtg tggagccagg cctggccagg caggggcctg 2640
aaacccactc ctccatctca tgggctcacg gcctacacga gcccacaagc tgccactggc 2700
cggcgacact gacacctgag cagtgtccag aacctttttg cctttttttg ttccccgtga 2760
aaagcaacat ggacatttcc ttctagtcct tccaaggagg ggagagaagt gtatgtgcat 2820
ttgtgtgtgt gtgtgtgtgt tgtgtgtgtg tgtgcgctaa gtgagaaaga gagcaggctc 2880
gggaggccct gcccagggta ggaggagctt cctgctttgc accatctggt ggtcgcaccc 2940

tggagggcac cccgactctg tctccaggag tctcatcagc aaaccgctga caagtctttc 3000

tagaaattct actgcactgc ctggctcagc tgcacgctgc agacatttct gcaggaggag 3060

caggtgtttc tgtcttctgt tccttctagg gccacctgtc cccttaaaca caggtccacg 3120

ttgtgtcaag aacctagtgc atctgtgtgt gtctgtcagt gtctctgtgt cagtgttctc 3180

gtgggtgtct gcacggtacc ggcccgcctt ctgcaatcat cactcccgca gagggggtgc 3240

agatcaggcg ccgtgctgcg gttgttgttc aacagtgctt tttcttagat agcgtcttcc 3300

tcagcgcccg tcggttgtgg catccttgat ctcagggatc ttctccgttt gcatgtcctc 3360

ggagtggcgt gttccttctc cctgggtccg acatgtgttc ccgcacctgc atggactgcc 3420

ccggttctgt gttgtgtgcc gagtgccgcc cagtgttctg tgaccacccg tgtagctact 3480

gaaaatggct gggtaagcaa gtcaagggtg ttggaggagg tcaagagaga gctcagtttc 3540

cctctccccc tccccaaaca caccaagaag catttttaac gtgtaggttg agaacaagcc 3600

taaaggattc ccacagctgg gagccagcaa gagagcttgg agtcgcctct ctagaccaga 3660

tctagcccca ccctcactcc agccatctcg gagcccttgt gtaggcaacg ccggtgcgcg 3720

gctgtgtggg gtgctccctg ccagcacctc cggccagccc cgcccctgcc gatctactgg 3780

accgcagacc accttctgcc cccgtggcca ggtgggagct gtccgttcag gaccatgagc 3840

catcctctgc cctgactagc gaggggcaga gcacacccca gtgcttacgc ctccacccct 3900

gcagcctcct ggcccgctca ccttcctcac ccctcctctg acccacccat ggtgccaggg 3960

ccgaagctga cctttagctc cctcctgccc ttgctagggt ctgagccaag cccctcgact 4020

cctcactgtg ttgacacttg gcactttgct ggccccgaga aggtcgatga cacagccgca 4080

aatctaatcc acgtagttcc catttactcc ttaatctgat tgatgttccc tcttgcactg 4140

aataatacat gcctctctca ggtaagccat ttttataaac aagaagataa aaagcactgt 4200

tgaggcagtg tttgcttttg ccgagctggt gtccgacagc tccctgggtg tccggggtgg 4260

gagagctgtt gacagaagct ctccgggcct cagggcttag atcccacttg agtcgtaagc 4320

cttcttgctt ttgataacac agtattattt ctcttactgt agaagaaaaa gtttattacc 4380

aaacaagagt atttttatga aagaaaagga caaacctata aattaactca acctatatct 4440

cccttgaaaa tactttcagg ctccaccaaa acgtagaact gaaagcatgt attttggaag 4500

aaagagatac attttgtatg ctttcttttc cttttgtaga ttcccagttt attttctaag 4560

actgcaaaga tcactttgtc accagccctg ggacctgaga ccaagggggt gtcttgtggg 4620

cagtgagggg gtgaggagag gctggcatga ggttcagtca ttccagtgag ctccaaagag 4680

gggccacctg ttctcaaaag catgttgggg accaggaggt aaaactggcc atttatggtg 4740

aacctgtgtc ttggagctga cttactaagt ggaatgagcc gaggatttga atatcagttc 4800

taaccttgat agaagaacct tgggttacat gtggttcaca ttaagaggat agaatccttt 4860

ggaatcttat ggcaaccaaa tgtggcttga cgaagtcgtg gtttcatctc ttaaacacag 4920

tgtgtaaatt tattcaacta acgatgggaa atgtattact tctgtacaca gtggactgaa 4980

gtgcaatttg ttgaaaggga acaagtcatt gaagagaaaa aaaaaaagcc caatacttag 5040

agtcccaatt ttgtctcatt tgccaaaaaa a 5071

<210> 12
<211> 430
<212> PRT
<213> Homo sapiens

<400> 12

Met Pro Ala Gly Arg Ala Arg Thr Cys Ala Leu Leu Ala Leu Cys Leu
1               5                   10                  15

Leu Gly Pro Gln Asp Phe Gly Pro Thr Arg Phe Ile Cys Thr Ser Val
            20                  25                  30

Pro Val Asp Ala Asp Met Cys Ala Ala Ser Val Ala Ala Gly Gly Ala
        35                  40                  45

Glu Glu Leu Arg Ser Ser Asn Val Leu Gln Leu Arg Glu Thr Val Leu
    50                  55                  60

Gln Gln Lys Glu Thr Ile Leu Ser Gln Lys Glu Thr Ile Arg Glu Leu
65                  70                  75                  80

Thr Ala Lys Leu Gly Arg Cys Glu Ser Gln Ser Thr Leu Asp Pro Gly

                85                  90                  95

Ala Gly Glu Ala Arg Ala Gly Gly Gly Arg Lys Gln Pro Gly Ser Gly
            100                 105                 110

Lys Asn Thr Met Gly Asp Leu Ser Arg Thr Pro Ala Ala Glu Thr Leu
        115                 120                 125

Ser Gln Leu Gly Gln Thr Leu Gln Ser Leu Lys Thr Arg Leu Glu Asn
    130                 135                 140

Leu Glu Gln Tyr Ser Arg Leu Asn Ser Ser Ser Gln Thr Asn Ser Leu
145                 150                 155                 160

Lys Asp Leu Leu Gln Ser Lys Ile Asp Glu Leu Glu Arg Gln Val Leu
                165                 170                 175

Ser Arg Val Asn Thr Leu Glu Glu Gly Lys Gly Gly Pro Lys Asn Asp
            180                 185                 190

Thr Glu Glu Arg Val Lys Ile Glu Thr Ala Leu Thr Ser Leu His Gln
        195                 200                 205

Arg Ile Ser Glu Leu Glu Lys Gly Gln Lys Asp Asn Arg Pro Gly Asp
    210                 215                 220

Lys Phe Gln Leu Thr Phe Pro Leu Arg Thr Asn Tyr Met Tyr Ala Lys
225                 230                 235                 240

Val Lys Lys Ser Leu Pro Glu Met Tyr Ala Phe Thr Val Cys Met Trp
                245                 250                 255

Leu Lys Ser Ser Ala Thr Pro Gly Val Gly Thr Pro Phe Ser Tyr Ala
            260                 265                 270

Val Pro Gly Gln Ala Asn Glu Leu Val Leu Ile Glu Trp Gly Asn Asn
        275                 280                 285

Pro Met Glu Ile Leu Ile Asn Asp Lys Val Ala Lys Leu Pro Phe Val
    290                 295                 300

Ile Asn Asp Gly Lys Trp His His Ile Cys Val Thr Trp Thr Thr Arg
305                 310                 315                 320

Asp Gly Val Trp Glu Ala Tyr Gln Asp Gly Thr Gln Gly Gly Ser Gly
                325                 330                 335

Glu Asn Leu Ala Pro Tyr His Pro Ile Lys Pro Gln Gly Val Leu Val
            340                 345                 350

Leu Gly Gln Glu Gln Asp Thr Leu Gly Gly Gly Phe Asp Ala Thr Gln
        355                 360                 365

Ala Phe Val Gly Glu Leu Ala His Phe Asn Ile Trp Asp Arg Lys Leu
    370                 375                 380

Thr Pro Gly Glu Val Tyr Asn Leu Ala Thr Cys Ser Thr Lys Ala Leu
385                 390                 395                 400

Ser Gly Asn Val Ile Ala Trp Ala Glu Ser His Ile Glu Ile Tyr Gly
                405                 410                 415

Gly Ala Thr Lys Trp Thr Phe Glu Ala Cys Arg Gln Ile Asn
            420                 425                 430

<210>  13
<211>  51
<212>  DNA
<213>  Artificial Sequence

<220>
<221>  source
<223>  /note=" Description of artificial sequence: An artificially
       synthesized sequence for siRNA"

<400>  13
tccccctgtg gcagtacaac aagttcaaga gacttgttgt actgccacag g                51

<210>  14

<211>  51
<212>  DNA
<213>  Artificial Sequence

<220>
<221>  source
<223>  /note=" Description of artificial sequence: An artificially
       synthesized sequence for siRNA"

<400>  14
aaaacctgtg gcagtacaac aagtctcttg aacttgttgt actgccacag g                51


<210>  15
<211>  51
<212>  DNA
<213>  Artificial Sequence

<220>
<221>  source
<223>  /note=" Description of artificial sequence: An artificially
       synthesized sequence for siRNA"

<400>  15
tccctgccag acaagaagtg gtgttcaaga gacaccactt cttgtctggc a                51


<210>  16
<211>  51
<212>  DNA
<213>  Artificial Sequence

<220>
<221>  source
<223>  /note=" Description of artificial sequence: An artificially
       synthesized sequence for siRNA"

<400>  16
aaaatgccag acaagaagtg gtgtctcttg aacaccactt cttgtctggc a                51


<210>  17
<211>  51
<212>  DNA
<213>  Artificial Sequence

<220>
<221>  source
<223>  /note=" Description of artificial sequence: An artificially
       synthesized sequence for siRNA"

<400> 17
tcccgatgct gctgaaaggg agattcaaga gatctccctt tcagcagcat c 51

<210> 18
<211> 51
<212> DNA
<213> Artificial Sequence

<220>
<221> source
<223> /note=" Description of artificial sequence: An artificially
synthesized sequence for siRNA"

<400> 18
aaaagatgct gctgaaaggg agatctcttg aatctccctt tcagcagcat c 51

<210> 19
<211> 51
<212> DNA
<213> Artificial Sequence

<220>
<221> source
<223> /note=" Description of artificial sequence: An artificially
synthesized sequence for siRNA"

<400> 19
tccccagcag aagctattca gacttcaaga gagtctgaat agcttctgct g 51

<210> 20
<211> 51
<212> DNA
<213> Artificial Sequence

<220>
<221> source
<223> /note=" Description of artificial sequence: An artificially
synthesized sequence for siRNA"

<400> 20
aaaacagcag aagctattca gactctcttg aagtctgaat agcttctgct g 51

<210> 21
<211> 51
<212> DNA
<213> Artificial Sequence

<220>
<221> source
<223> /note=" Description of artificial sequence: An artificially
      synthesized sequence for siRNA"

<400> 21
tcccggtgtc ctccatccaa gaattcaaga gattcttgga tggaggacac c          51


<210> 22
<211> 51
<212> DNA
<213> Artificial Sequence

<220>
<221> source
<223> /note=" Description of artificial sequence: An artificially
      synthesized sequence for siRNA"

<400> 22
aaaaggtgtc ctccatccaa gaatctcttg aattcttgga tggaggacac c          51


<210> 23
<211> 51
<212> DNA
<213> Artificial Sequence

<220>
<221> source
<223> /note=" Description of artificial sequence: An artificially
      synthesized sequence for siRNA"

<400> 23
tcccgccgtg ctaacactgt tacttcaaga gagtaacagt gttagcacgg c          51


<210> 24
<211> 51
<212> DNA
<213> Artificial Sequence

<220>
<221> source
<223> /note=" Description of artificial sequence: An artificially
      synthesized sequence for siRNA"

<400> 24
aaaagccgtg ctaacactgt tactctcttg aagtaacagt gttagcacgg c          51

<210>  25
<211>  51
<212>  DNA
<213>  Artificial Sequence

<220>
<221>  source
<223>  /note=" Description of artificial sequence: An artificially
       synthesized sequence for siRNA"

<400>  25
tcccgaagct ctccaaccgt ctcttcaaga gagagacggt tggagagctt c                51


<210>  26
<211>  51
<212>  DNA
<213>  Artificial Sequence

<220>
<221>  source
<223>  /note=" Description of artificial sequence: An artificially
       synthesized sequence for siRNA"

<400>  26
aaaagaagct ctccaaccgt ctctctcttg aagagacggt tggagagctt c                51


<210>  27
<211>  51
<212>  DNA
<213>  Artificial Sequence

<220>
<221>  source
<223>  /note=" Description of artificial sequence: An artificially
       synthesized sequence for siRNA"

<400>  27
tcccgactca gtacctcgcc ttgttcaaga gacaaggcga ggtactgagt c                51


<210>  28
<211>  51
<212>  DNA
<213>  Artificial Sequence

<220>
<221>  source
<223>  /note=" Description of artificial sequence: An artificially
       synthesized sequence for siRNA"

<400> 28
aaaagactca gtacctcgcc ttgtctcttg aacaaggcga ggtactgagt c 51

<210> 29
<211> 51
<212> DNA
<213> Artificial Sequence

<220>
<221> source
<223> /note=" Description of artificial sequence: An artificially
synthesized sequence for siRNA"

<400> 29
tcccggtttc agaagactca gtattcaaga gatactgagt cttctgaaac c 51

<210> 30
<211> 51
<212> DNA
<213> Artificial Sequence

<220>
<221> source
<223> /note=" Description of artificial sequence: An artificially
synthesized sequence for siRNA"

<400> 30
aaaaggtttc agaagactca gtatctcttg aatactgagt cttctgaaac c 51

<210> 31
<211> 51
<212> DNA
<213> Artificial Sequence

<220>
<221> source
<223> /note=" Description of artificial sequence: An artificially
synthesized sequence for siRNA"

<400> 31
tcccgtgcag ccagctcaat caattcaaga gattgattga gctggctgca c 51

<210> 32
<211> 51
<212> DNA
<213> Artificial Sequence

<220>
<221>  source
<223>  /note=" Description of artificial sequence: An artificially
      synthesized sequence for siRNA"

<400>  32
aaaagtgcag ccagctcaat caatctcttg aattgattga gctggctgca c                51


<210>  33
<211>  51
<212>  DNA
<213>  Artificial Sequence

<220>
<221>  source
<223>  /note=" Description of artificial sequence: An artificially
      synthesized sequence for siRNA"

<400>  33
tcccgagaat tcattactac agcttcaaga gagctgtagt aatgaattct c                51


<210>  34
<211>  51
<212>  DNA
<213>  Artificial Sequence

<220>
<221>  source
<223>  /note=" Description of artificial sequence: An artificially
      synthesized sequence for siRNA"

<400>  34
aaaagagaat tcattactac agctctcttg aagctgtagt aatgaattct c                51


<210>  35
<211>  51
<212>  DNA
<213>  Artificial Sequence

<220>
<221>  source
<223>  /note=" Description of artificial sequence: An artificially
      synthesized sequence for siRNA"

<400>  35
tcccggatat tcctgctgtt ccattcaaga gatggaacag caggaatatc c                51

<210> 36
<211> 51
<212> DNA
<213> Artificial Sequence

<220>
<221> source
<223> /note=" Description of artificial sequence: An artificially
      synthesized sequence for siRNA"

<400> 36
aaaaggatat tcctgctgtt ccatctcttg aatggaacag caggaatatc c          51


<210> 37
<211> 51
<212> DNA
<213> Artificial Sequence

<220>
<221> source
<223> /note=" Description of artificial sequence: An artificially
      synthesized sequence for siRNA"

<400> 37
tcccgatatt caggagcagc atgttcaaga gacatgctgc tcctgaatat c          51


<210> 38
<211> 51
<212> DNA
<213> Artificial Sequence

<220>
<221> source
<223> /note=" Description of artificial sequence: An artificially
      synthesized sequence for siRNA"

<400> 38
aaaagatatt caggagcagc atgtctcttg aacatgctgc tcctgaatat c          51


<210> 39
<211> 51
<212> DNA
<213> Artificial Sequence

<220>
<221> source
<223> /note=" Description of artificial sequence: An artificially

synthesized sequence for siRNA"

<400> 39
tcccggagac catcctgagc cagttcaaga gactggctca ggatggtctc c 51

<210> 40
<211> 51
<212> DNA
<213> Artificial Sequence

<220>
<221> source
<223> /note=" Description of artificial sequence: An artificially
synthesized sequence for siRNA"

<400> 40
aaaaggagac catcctgagc cagtctcttg aactggctca ggatggtctc c 51

<210> 41
<211> 51
<212> DNA
<213> Artificial Sequence

<220>
<221> source
<223> /note=" Description of artificial sequence: An artificially
synthesized sequence for siRNA"

<400> 41
tcccgtggac cttcgaggcc tgtttcaaga gaacaggcct cgaaggtcca c 51

<210> 42
<211> 51
<212> DNA
<213> Artificial Sequence

<220>
<221> source
<223> /note=" Description of artificial sequence: An artificially
synthesized sequence for siRNA"

<400> 42
aaaagtggac cttcgaggcc tgttctcttg aaacaggcct cgaaggtcca c 51

<210> 43
<211> 47
<212> DNA

<213>  Artificial Sequence

<220>
<221>  source
<223>  /note=" Description of artificial sequence: siRNA hairpin design"

<400>  43
cctgtggcag tacaacaagt tcaagagact tgttgtactg ccacagg                    47


<210>  44
<211>  47
<212>  DNA
<213>  Artificial Sequence

<220>
<221>  source
<223>  /note=" Description of artificial sequence: siRNA hairpin design"

<400>  44
tgccagacaa gaagtggtgt tcaagagaca ccacttcttg tctggca                    47


<210>  45
<211>  47
<212>  DNA
<213>  Artificial Sequence

<220>
<221>  source
<223>  /note=" Description of artificial sequence: siRNA hairpin design"

<400>  45
gatgctgctg aaagggagat tcaagagatc tccctttcag cagcatc                    47


<210>  46
<211>  47
<212>  DNA
<213>  Artificial Sequence

<220>
<221>  source
<223>  /note=" Description of artificial sequence: siRNA hairpin design"

<400>  46
cagcagaagc tattcagact tcaagagagt ctgaatagct tctgctg                    47


<210>  47
<211>  47

<212>  DNA
<213>  Artificial Sequence

<220>
<221>  source
<223>  /note=" Description of artificial sequence: siRNA hairpin design"

<400>  47
ggtgtcctcc atccaagaat tcaagagatt cttggatgga ggacacc                47


<210>  48
<211>  47
<212>  DNA
<213>  Artificial Sequence

<220>
<221>  source
<223>  /note=" Description of artificial sequence: siRNA hairpin design"

<400>  48
gccgtgctaa cactgttact tcaagagagt aacagtgtta gcacggc                47


<210>  49
<211>  47
<212>  DNA
<213>  Artificial Sequence

<220>
<221>  source
<223>  /note=" Description of artificial sequence: siRNA hairpin design"

<400>  49
gaagctctcc aaccgtctct tcaagagaga gacggttgga gagcttc                47


<210>  50
<211>  47
<212>  DNA
<213>  Artificial Sequence

<220>
<221>  source
<223>  /note=" Description of artificial sequence: siRNA hairpin design"

<400>  50
gactcagtac ctcgccttgt tcaagagaca aggcgaggta ctgagtc                47


<210>  51

<211>  47
<212>  DNA
<213>  Artificial Sequence

<220>
<221>  source
<223>  /note=" Description of artificial sequence: siRNA hairpin design"

<400>  51
ggtttcagaa gactcagtat tcaagagata ctgagtcttc tgaaacc                    47


<210>  52
<211>  47
<212>  DNA
<213>  Artificial Sequence

<220>
<221>  source
<223>  /note=" Description of artificial sequence: siRNA hairpin design"

<400>  52
gtgcagccag ctcaatcaat tcaagagatt gattgagctg gctgcac                    47


<210>  53
<211>  47
<212>  DNA
<213>  Artificial Sequence

<220>
<221>  source
<223>  /note=" Description of artificial sequence: siRNA hairpin design"

<400>  53
gagaattcat tactacagct tcaagagagc tgtagtaatg aattctc                    47


<210>  54
<211>  47
<212>  DNA
<213>  Artificial Sequence

<220>
<221>  source
<223>  /note=" Description of artificial sequence: siRNA hairpin design"

<400>  54
ggatattcct gctgttccat tcaagagatg gaacagcagg aatatcc                    47

<210>  55
<211>  47
<212>  DNA
<213>  Artificial Sequence

<220>
<221>  source
<223>  /note=" Description of artificial sequence: siRNA hairpin design"

<400>  55
gatattcagg agcagcatgt tcaagagaca tgctgctcct gaatatc                47


<210>  56
<211>  47
<212>  DNA
<213>  Artificial Sequence

<220>
<221>  source
<223>  /note=" Description of artificial sequence: siRNA hairpin design"

<400>  56
ggagaccatc ctgagccagt tcaagagact ggctcaggat ggtctcc                47


<210>  57
<211>  47
<212>  DNA
<213>  Artificial Sequence

<220>
<221>  source
<223>  /note=" Description of artificial sequence: siRNA hairpin design"

<400>  57
gtggaccttc gaggcctgtt tcaagagaac aggcctcgaa ggtccac                47


<210>  58
<211>  19
<212>  DNA
<213>  Artificial Sequence

<220>
<221>  source
<223>  /note=" Description of artificial sequence: An artificially
       synthesized target sequence for siRNA"

<400>  58
cctgtggcag tacaacaag                                               19

<210> 59
<211> 19
<212> DNA
<213> Artificial Sequence

<220>
<221> source
<223> /note=" Description of artificial sequence: An artificially
      synthesized target sequence for siRNA"

<400> 59
tgccagacaa gaagtggtg                                                    19


<210> 60
<211> 19
<212> DNA
<213> Artificial Sequence

<220>
<221> source
<223> /note=" Description of artificial sequence: An artificially
      synthesized target sequence for siRNA"

<400> 60
gatgctgctg aaagggaga                                                    19


<210> 61
<211> 19
<212> DNA
<213> Artificial Sequence

<220>
<221> source
<223> /note=" Description of artificial sequence: An artificially
      synthesized target sequence for siRNA"

<400> 61
cagcagaagc tattcagac                                                    19


<210> 62
<211> 19
<212> DNA
<213> Artificial Sequence

<220>
<221> source

<223>  /note=" Description of artificial sequence: An artificially
      synthesized target sequence for siRNA"

<400>  62
ggtgtcctcc atccaagaa                                                    19


<210>  63
<211>  19
<212>  DNA
<213>  Artificial Sequence

<220>
<221>  source
<223>  /note=" Description of artificial sequence: An artificially
      synthesized target sequence for siRNA"

<400>  63
gccgtgctaa cactgttac                                                    19


<210>  64
<211>  19
<212>  DNA
<213>  Artificial Sequence

<220>
<221>  source
<223>  /note=" Description of artificial sequence: An artificially
      synthesized target sequence for siRNA"

<400>  64
gaagctctcc aaccgtctc                                                    19


<210>  65
<211>  19
<212>  DNA
<213>  Artificial Sequence

<220>
<221>  source
<223>  /note=" Description of artificial sequence: An artificially
      synthesized target sequence for siRNA"

<400>  65
gactcagtac ctcgccttg                                                    19


<210>  66
<211>  19

<212>  DNA
<213>  Artificial Sequence

<220>
<221>  source
<223>  /note=" Description of artificial sequence: An artificially
       synthesized target sequence for siRNA"

<400>  66
ggtttcagaa gactcagta                                                    19


<210>  67
<211>  19
<212>  DNA
<213>  Artificial Sequence

<220>
<221>  source
<223>  /note=" Description of artificial sequence: An artificially
       synthesized target sequence for siRNA"

<400>  67
gtgcagccag ctcaatcaa                                                    19


<210>  68
<211>  19
<212>  DNA
<213>  Artificial Sequence

<220>
<221>  source
<223>  /note=" Description of artificial sequence: An artificially
       synthesized target sequence for siRNA"

<400>  68
gagaattcat tactacagc                                                    19


<210>  69
<211>  19
<212>  DNA
<213>  Artificial Sequence

<220>
<221>  source
<223>  /note=" Description of artificial sequence: An artificially
       synthesized target sequence for siRNA"

<400>  69

ggatattcct gctgttcca                                                    19

<210>  70
<211>  19
<212>  DNA
<213>  Artificial Sequence

<220>
<221>  source
<223>  /note=" Description of artificial sequence: An artificially
       synthesized target sequence for siRNA"

<400>  70
gatattcagg agcagcatg                                                    19

<210>  71
<211>  19
<212>  DNA
<213>  Artificial Sequence

<220>
<221>  source
<223>  /note=" Description of artificial sequence: An artificially
       synthesized target sequence for siRNA"

<400>  71
ggagaccatc ctgagccag                                                    19

<210>  72
<211>  19
<212>  DNA
<213>  Artificial Sequence

<220>
<221>  source
<223>  /note=" Description of artificial sequence: An artificially
       synthesized target sequence for siRNA"

<400>  72
gtggaccttc gaggcctgt                                                    19

<210>  73
<211>  22
<212>  DNA
<213>  Artificial Sequence

<220>

<221>  source
<223>  /note=" Description of artificial sequence: an artificially
      synthesized primer sequence for RT-PCR"

<400>  73
atgcaggacg gtaacttcct gc                                              22


<210>  74
<211>  21
<212>  DNA
<213>  Artificial Sequence

<220>
<221>  source
<223>  /note=" Description of artificial sequence: an artificially
      synthesized primer sequence for RT-PCR"

<400>  74
tgggcccagg aagtcctcct t                                               21


<210>  75
<211>  23
<212>  DNA
<213>  Artificial Sequence

<220>
<221>  source
<223>  /note=" Description of artificial sequence: an artificially
      synthesized primer sequence for RT-PCR"

<400>  75
gagaaactga agtcccagga aat                                             23


<210>  76
<211>  23
<212>  DNA
<213>  Artificial Sequence

<220>
<221>  source
<223>  /note=" Description of artificial sequence: an artificially
      synthesized primer sequence for RT-PCR"

<400>  76
ctgatacttc cattcgcttc aac                                             23


<210>  77

<211>  23
<212>  DNA
<213>  Artificial Sequence

<220>
<221>  source
<223>  /note=" Description of artificial sequence: an artificially
       synthesized primer sequence for RT-PCR"

<400>  77
catctggcat ttctgctctc tat                                              23


<210>  78
<211>  25
<212>  DNA
<213>  Artificial Sequence

<220>
<221>  source
<223>  /note=" Description of artificial sequence: an artificially
       synthesized primer sequence for RT-PCR"

<400>  78
ctcagggaaa ggagaataaa agaac                                            25


<210>  79
<211>  23
<212>  DNA
<213>  Artificial Sequence

<220>
<221>  source
<223>  /note=" Description of artificial sequence: an artificially
       synthesized primer sequence for RT-PCR"

<400>  79
gggactactg tgttttgctg ttc                                              23


<210>  80
<211>  23
<212>  DNA
<213>  Artificial Sequence

<220>
<221>  source
<223>  /note=" Description of artificial sequence: an artificially
       synthesized primer sequence for RT-PCR"

<400>  80
tgaggtcata gatttcaagg cac                                                23

<210>  81
<211>  23
<212>  DNA
<213>  Artificial Sequence

<220>
<221>  source
<223>  /note=" Description of artificial sequence: an artificially
       synthesized primer sequence for RT-PCR"

<400>  81
ctaggaagaa atcatgctgg gtt                                                23

<210>  82
<211>  23
<212>  DNA
<213>  Artificial Sequence

<220>
<221>  source
<223>  /note=" Description of artificial sequence: an artificially
       synthesized primer sequence for RT-PCR"

<400>  82
gtatttggct tactgtccca aac                                                23

<210>  83
<211>  23
<212>  DNA
<213>  Artificial Sequence

<220>
<221>  source
<223>  /note=" Description of artificial sequence: an artificially
       synthesized primer sequence for RT-PCR"

<400>  83
taaccttgat agaagaacct tgg                                                23

<210>  84
<211>  22
<212>  DNA
<213>  Artificial Sequence

<220>
<221>  source
<223>  /note=" Description of artificial sequence: an artificially
      synthesized primer sequence for RT-PCR"

<400>  84
gcaaatgaga caaaattggg ac                                              22


<210>  85
<211>  22
<212>  DNA
<213>  Artificial Sequence

<220>
<221>  source
<223>  /note=" Description of artificial sequence: an artificially
      synthesized primer sequence"

<400>  85
tggtagccaa gtgcaggtta ta                                              22


<210>  86
<211>  22
<212>  DNA
<213>  Artificial Sequence

<220>
<221>  source
<223>  /note=" Description of artificial sequence: an artificially
      synthesized primer sequence"

<400>  86
ccaaagggtt tctgcagttt ca                                              22


<210>  87
<211>  30
<212>  DNA
<213>  Artificial Sequence

<220>
<221>  source
<223>  /note=" Description of artificial sequence: an artificially
      synthesized primer sequence"

<400>  87
tgcggatcca gagcagattg tactgagagt                                      30

<210> 88
<211> 29
<212> DNA
<213> Artificial Sequence

<220>
<221> source
<223> /note=" Description of artificial sequence: an artificially
synthesized primer sequence"

<400> 88
ctctatctcg agtgaggcgg aaagaacca 29

<210> 89
<211> 47
<212> DNA
<213> Artificial Sequence

<220>
<221> source
<223> /note=" Description of artificial sequence: an artificially
synthesized primer sequence"

<400> 89
tttaagcttg aagaccattt ttggaaaaaa aaaaaaaaaa aaaaaac 47

<210> 90
<211> 34
<212> DNA
<213> Artificial Sequence

<220>
<221> source
<223> /note=" Description of artificial sequence: an artificially
synthesized primer sequence"

<400> 90
tttaagcttg aagacatggg aaagagtggt ctca 34

<210> 91
<211> 5089
<212> DNA
<213> Artificial Sequence

<220>
<221> source
<223> /note=" Description of artificial sequence: an artificially
synthesized plasmid sequence"

<220>
<221>  misc_feature
<222>  (489)..(492)
<223>  "n" indicates GAP.

<400>  91
gacggatcgg gagatctccc gatcccctat ggtgcactct cagtacaatc tgctctggat      60

ccactagtaa cggccgccag tgtgctggaa ttcggcttgg tagccaagtg caggttatag     120

ggagctgaag ggaagggggt cacagtaggt ggcatcgttc ctttctgact gcccgccccc     180

cgcatgccgt cccgcgatat tgagctccga acctctcgcc ctgccgccgc cggtgctccg     240

tcgccgccgc gccgccatgg aattcgaacg ctgacgtcat caacccgctc caaggaatcg     300

cgggcccagt gtcactaggc gggaacaccc agcgcgcgtg cgccctggca ggaagatggc     360

tgtgagggac aggggagtgg cgccctgcaa tatttgcatg tcgctatgtg ttctgggaaa     420

tcaccataaa cgtgaaatgt ctttggattt gggaatctta taagttctgt atgagaccac     480

tctttcccnn nntttttggg aaaaaaaaaa aaaaaaaaaa aacgaaaccg ggccgggcgc     540

ggtggttcac gcctataatc ccagcacttt gggaggccga ggcgggcgga tcacaaggtc     600

aggaggtcga gaccatccag gctaacacgg tgaaaccccc ccccatctct actaaaaaaa     660

aaaaatacaa aaaattagcc attagccggg cgtggtggcg ggcgcctata atcccagcta     720

cttgggaggc tgaagcagaa tggcgtgaac ccgggaggcg gacgttgcag tgagccgaga     780

tcgcgccgac tgcattccag cctgggcgac agagcgagtc tcaaaaaaaa aaccgagtgg     840

aatgtgaaaa gctccgtgaa actgcagaaa cccaagccga attctgcaga tatccatcac     900

actggcggcc gctcgagtga ggcggaaaga accagctggg gctctagggg gtatccccac     960

gcgccctgta gcggcgcatt aagcgcggcg ggtgtggtgg ttacgcgcag cgtgaccgct    1020

acacttgcca gcgccctagc gcccgctcct ttcgctttct tcccttcctt tctcgccacg    1080

ttcgccggct ttccccgtca agctctaaat cgggggctcc ctttagggtt ccgatttagt    1140

gctttacggc acctcgaccc caaaaaactt gattagggtg atggttcacg tagtgggcca    1200

tcgccctgat agacggtttt tcgccctttg acgttggagt ccacgttctt taatagtgga    1260

ctcttgttcc aaactggaac aacactcaac cctatctcgg tctattcttt tgatttataa    1320

gggattttgc cgatttcggc ctattggtta aaaaatgagc tgatttaaca aaaatttaac 1380

gcgaattaat tctgtggaat gtgtgtcagt tagggtgtgg aaagtcccca ggctccccag 1440

caggcagaag tatgcaaagc atgcatctca attagtcagc aaccaggtgt ggaaagtccc 1500

caggctcccc agcaggcaga agtatgcaaa gcatgcatct caattagtca gcaaccatag 1560

tcccgcccct aactccgccc atcccgcccc taactccgcc cagttccgcc cattctccgc 1620

cccatggctg actaattttt tttatttatg cagaggccga ggccgcctct gcctctgagc 1680

tattccagaa gtagtgagga ggcttttttg gaggcctagg cttttgcaaa aagctcccgg 1740

gagcttgtat atccattttc ggatctgatc aagagacagg atgaggatcg tttcgcatga 1800

ttgaacaaga tggattgcac gcaggttctc cggccgcttg ggtggagagg ctattcggct 1860

atgactgggc acaacagaca atcggctgct ctgatgccgc cgtgttccgg ctgtcagcgc 1920

aggggcgccc ggttcttttt gtcaagaccg acctgtccgg tgccctgaat gaactgcagg 1980

acgaggcagc gcggctatcg tggctggcca cgacgggcgt tccttgcgca gctgtgctcg 2040

acgttgtcac tgaagcggga agggactggc tgctattggg cgaagtgccg gggcaggatc 2100

tcctgtcatc tcaccttgct cctgccgaga aagtatccat catggctgat gcaatgcggc 2160

ggctgcatac gcttgatccg gctacctgcc cattcgacca ccaagcgaaa catcgcatcg 2220

agcgagcacg tactcggatg gaagccggtc ttgtcgatca ggatgatctg gacgaagagc 2280

atcaggggct cgcgccagcc gaactgttcg ccaggctcaa ggcgcgcatg cccgacggcg 2340

aggatctcgt cgtgacccat ggcgatgcct gcttgccgaa tatcatggtg gaaaatggcc 2400

gcttttctgg attcatcgac tgtggccggc tgggtgtggc ggaccgctat caggacatag 2460

cgttggctac ccgtgatatt gctgaagagc ttggcggcga atgggctgac cgcttcctcg 2520

tgctttacgg tatcgccgct cccgattcgc agcgcatcgc cttctatcgc cttcttgacg 2580

agttcttctg agcgggactc tggggttcga aatgaccgac caagcgacgc ccaacctgcc 2640

atcacgagat ttcgattcca ccgccgcctt ctatgaaagg ttgggcttcg gaatcgtttt 2700

ccgggacgcc ggctggatga tcctccagcg cggggatctc atgctggagt tcttcgccca 2760

ccccaacttg tttattgcag cttataatgg ttacaaataa agcaatagca tcacaaattt 2820

cacaaataaa gcattttttt cactgcattc tagttgtggt ttgtccaaac tcatcaatgt 2880

atcttatcat gtctgtatac cgtcgacctc tagctagagc ttggcgtaat catggtcata 2940

gctgtttcct gtgtgaaatt gttatccgct cacaattcca cacaacatac gagccggaag 3000

cataaagtgt aaagcctggg gtgcctaatg agtgagctaa ctcacattaa ttgcgttgcg 3060

ctcactgccc gctttccagt cgggaaacct gtcgtgccag ctgcattaat gaatcggcca 3120

acgcgcgggg agaggcggtt tgcgtattgg gcgctcttcc gcttcctcgc tcactgactc 3180

gctgcgctcg gtcgttcggc tgcggcgagc ggtatcagct cactcaaagg cggtaatacg 3240

gttatccaca gaatcagggg ataacgcagg aaagaacatg tgagcaaaag gccagcaaaa 3300

ggccaggaac cgtaaaaagg ccgcgttgct ggcgtttttc cataggctcc gcccccctga 3360

cgagcatcac aaaaatcgac gctcaagtca gaggtggcga aacccgacag gactataaag 3420

ataccaggcg tttccccctg gaagctccct cgtgcgctct cctgttccga ccctgccgct 3480

taccggatac ctgtccgcct ttctcccttc gggaagcgtg gcgctttctc atagctcacg 3540

ctgtaggtat ctcagttcgg tgtaggtcgt tcgctccaag ctgggctgtg tgcacgaacc 3600

ccccgttcag cccgaccgct gcgccttatc cggtaactat cgtcttgagt ccaacccggt 3660

aagacacgac ttatcgccac tggcagcagc cactggtaac aggattagca gagcgaggta 3720

tgtaggcggt gctacagagt tcttgaagtg gtggcctaac tacggctaca ctagaagaac 3780

agtatttggt atctgcgctc tgctgaagcc agttaccttc ggaaaaagag ttggtagctc 3840

ttgatccggc aaacaaacca ccgctggtag cggttttttt gtttgcaagc agcagattac 3900

gcgcagaaaa aaaggatctc aagaagatcc tttgatcttt tctacggggt ctgacgctca 3960

gtggaacgaa aactcacgtt aagggatttt ggtcatgaga ttatcaaaaa ggatcttcac 4020

ctagatcctt ttaaattaaa aatgaagttt taaatcaatc taaagtatat atgagtaaac 4080

ttggtctgac agttaccaat gcttaatcag tgaggcacct atctcagcga tctgtctatt 4140

tcgttcatcc atagttgcct gactccccgt cgtgtagata actacgatac gggagggctt 4200

accatctggc cccagtgctg caatgatacc gcgagaccca cgctcaccgg ctccagattt 4260

atcagcaata aaccagccag ccggaagggc cgagcgcaga agtggtcctg caactttatc 4320

cgcctccatc cagtctatta attgttgccg ggaagctaga gtaagtagtt cgccagttaa 4380

tagtttgcgc aacgttgttg ccattgctac aggcatcgtg gtgtcacgct cgtcgtttgg 4440

tatggcttca ttcagctccg gttcccaacg atcaaggcga gttacatgat cccccatgtt 4500

gtgcaaaaaa gcggttagct ccttcggtcc tccgatcgtt gtcagaagta agttggccgc 4560

agtgttatca ctcatggtta tggcagcact gcataattct cttactgtca tgccatccgt 4620

aagatgcttt tctgtgactg gtgagtactc aaccaagtca ttctgagaat agtgtatgcg 4680

gcgaccgagt tgctcttgcc cggcgtcaat acgggataat accgcgccac atagcagaac 4740

tttaaaagtg ctcatcattg gaaaacgttc ttcggggcga aaactctcaa ggatcttacc 4800

gctgttgaga tccagttcga tgtaacccac tcgtgcaccc aactgatctt cagcatcttt 4860

tactttcacc agcgtttctg ggtgagcaaa aacaggaagg caaaatgccg caaaaaaggg 4920

aataagggcg acacggaaat gttgaatact catactcttc ctttttcaat attattgaag 4980

catttatcag ggttattgtc tcatgagcgg atacatattt gaatgtattt agaaaaataa 5040

acaaataggg gttccgcgca catttccccg aaaagtgcca cctgacgtc 5089

<210> 92
<211> 51
<212> DNA
<213> Artificial Sequence

<220>
<221> source
<223> /note=" Description of artificial sequence: An artificially
      synthesized sequence for siRNA"

<400> 92
caccgaagcagcacgacttcttcttcaagagagaagaagtcgtgctgcttc 51

<210> 93
<211> 51
<212> DNA
<213> Artificial Sequence

<220>
<221> source
<223> /note=" Description of artificial sequence: An artificially
      synthesized sequence for siRNA"

<400> 93

aaaagaagca gcacgacttc ttctctcttg aagaagaagt cgtgctgctt c          51


<210>  94
<211>  51
<212>  DNA
<213>  Artificial Sequence

<220>
<221>  source
<223>  /note=" Description of artificial sequence: An artificially
       synthesized sequence for siRNA"

<400>  94
tccccgtacg cggaatactt cgattcaaga gatcgaagta ttccgcgtac g          51


<210>  95
<211>  51
<212>  DNA
<213>  Artificial Sequence

<220>
<221>  source
<223>  /note=" Description of artificial sequence: An artificially
       synthesized sequence for siRNA"

<400>  95
aaaacgtacg cggaatactt cgatctcttg aatcgaagta ttccgcgtac g          51


<210>  96
<211>  51
<212>  DNA
<213>  Artificial Sequence

<220>
<221>  source
<223>  /note=" Description of artificial sequence: An artificially
       synthesized sequence for siRNA"

<400>  96
tcccgcgcgc tttgtaggat tcgttcaaga gacgaatcct acaaagcgcg c          51


<210>  97
<211>  51
<212>  DNA
<213>  Artificial Sequence

<220>

<221>  source
<223>  /note=" Description of artificial sequence: An artificially
      synthesized sequence for siRNA"

<400>  97
aaaagcgcgc tttgtaggat tcgtctcttg aacgaatcct acaaagcgcg c                51


<210>  98
<211>  47
<212>  DNA
<213>  Artificial Sequence

<220>
<221>  source
<223>  /note=" Description of artificial sequence: siRNA hairpin design"

<400>  98
gaagcagcac gacttcttct tcaagagaga agaagtcgtg ctgcttc                     47


<210>  99
<211>  47
<212>  DNA
<213>  Artificial Sequence

<220>
<221>  source
<223>  /note=" Description of artificial sequence: siRNA hairpin design"

<400>  99
cgtacgcgga atacttcgat tcaagagatc gaagtattcc gcgtacg                     47


<210>  100
<211>  47
<212>  DNA
<213>  Artificial Sequence

<220>
<221>  source
<223>  /note=" Description of artificial sequence: siRNA hairpin design"

<400>  100
gcgcgctttg taggattcgt tcaagagacg aatcctacaa agcgcgc                     47


<210>  101
<211>  19
<212>  DNA
<213>  Artificial Sequence

<220>
<221> source
<223> /note=" Description of artificial sequence: An artificially
      synthesized target sequence for siRNA"

<400> 101
gaagcagcac gacttcttc                                                    19


<210> 102
<211> 19
<212> DNA
<213> Artificial Sequence

<220>
<221> source
<223> /note=" Description of artificial sequence: An artificially
      synthesized target sequence for siRNA"

<400> 102
cgtacgcgga atacttcga                                                    19


<210> 103
<211> 19
<212> DNA
<213> Artificial Sequence

<220>
<221> source
<223> /note=" Description of artificial sequence: An artificially
      synthesized target sequence for siRNA"

<400> 103
gcgcgctttg taggattcg                                                    19

## Description

This application claims the benefit of U.S. Provisional Application Serial No.60/555,757 filed March 24, 2004, the contents of which are hereby incorporated by reference in its entirety.

### Technical Field

The present invention relates to the field of biological science, more specifically to the field of cancer research. In particular, the present invention relates a composition comprising a nucleic acid capable of inhibiting expression of PKP3, CDCA1, CDCA8, DLX5, URLC11 or NPTX1. In some embodiments, the nucleic acid is a small interfering RNA (siRNA) corresponding to a subsequence from these genes.

### Background Art

Lung cancer is one of the most common cancers in the world. Non-small cell lung cancer (NSCLC) is by far the most common form, accounting for nearly 80% of lung tumors (American Cancer Society. Cancer Facts and Figures 2001 (Am. Chem. Soc. Atlanta). 2001). The overall 10-year survival rate remains as low as 10% despite recent advances in multi-modality therapy, because the majority of NSCLCs are not diagnosed until advanced stages (Fry, W. A., Phillips, J. L., and Menck, H. R. Ten-year survey of lung cancer treatment and survival in hospitals in the United States: a national cancer data base report, Cancer. 86: 1867-76, 1999.). Although chemotherapy regimens based on platinum are considered the reference standards for treatment of NSCLC, those drugs are able to extend survival of patients with advanced NSCLC only about six weeks (Chemotherapy in non-small cell lung cancer: a meta-analysis using updated data on individual patients from 52 randomised clinical trials. Non-small Cell Lung Cancer Collaborative Group, Bmj. 311: 899-909, 1995.). Numerous targeted therapies are being investigated for this disease, including tyrosine kinase inhibitors, but so far promising results have been achieved in only a limited number of patients and some recipients suffer severe adverse reactions (Kris M, N. R., Herbst RS A phase II trial of ZD1839 ('Iressa') in advanced non-small cell lung cancer (NSCLC) patients who had failed platinum- and docetaxel-based regimens (IDEAL 2), Proc Am Soc Clin Oncol. 21: 292a(A1166), 2002.). Therefore development of highly effective chemotherapeutic drugs and preventive strategies are matters of pressing concern.

### Disclosure of the Invention

The present invention based on the surprising discovery that inhibiting expression of PKP3, CDCA1, CDCA8, DLX5, URLC11 or NPTX1 is effective in inhibiting the cellular growth of various cancer cells, including those involved in NSCLC. The inventions described in this application are based in part on this discovery.

The invention provides methods for inhibiting cell growth. Among the methods provided are those comprising contacting a cell with a composition comprising a small interfering RNA (siRNA) that inhibits expression of PKP3, CDCA1, CDCA8, DLX5, URLC11 or NPTX1. The invention also provides methods for inhibiting tumor cell growth in a subject. Such methods include administering to a subject a composition comprising a small interfering RNA (siRNA) that hybridizes specifically to a sequence from PKF3, CDCA1, CDCA8, DLX5, URLC11 or NPTX1. Another aspect of the invention provides methods for inhibiting the expression of the PKP3, CDCA1, CDCA8, DLX5, URLC11 or NPTX1 gene in a cell of a biological sample. Expression of the gene may be inhibited by introduction of a double stranded ribonucleic acid (RNA) molecule into the cell in an amount sufficient to inhibit expression of the PKP3, CDCA1., CDCA8, DLX5, URLC11 or NPTX1 gene. Another aspect of the invention relates to products including nucleic acid sequences and vectors as well as to compositions comprising them, useful, for example, in the provided methods. Among the products provided are siRNA molecules having the property to inhibit expression of the PKP3, CDCA1, CDCA8, DLX5, URLC11 or NPTX1 gene when introduced into a cell expressing said gene. Among such molecules are those that comprise a sense strand and an antisense strand, wherein the sense strand comprises a ribonucleotide sequence corresponding to a PKP3, CDCA1, CDCA8, DLX5, URLC11 or NPTX1 target sequence, and wherein the antisense strand comprises a ribonucleotide sequence which is complementary to said sense strand. The sense and the antisense strands of the molecule hybridize to each other to form a double-stranded molecule.

As used herein, the term "organism" refers to any living entity comprised of at least one cell. A living organism can be as simple as, for example, a single eukaryotic cell or as complex as a mammal, including a human being.

As used herein, the term "biological sample" refers to a whole organism or a subset of its tissues, cells or component parts (e.g. bodily fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate, extract, cell culture or tissue culture prepared from a whole organism or a subset of its cells, tissues or component parts, or a fraction or portion thereof. Lastly, "biological sample" refers to a medium, such as a nutrient broth or gel in which an organism has been propagated, which contains cellular components, such as proteins or nucleic acid molecules.

The invention features methods of inhibiting cell growth. Cell growth is inhibited by contacting a cell with a composition of a small interfering RNA (siRNA) of PKP3, CDCA1, CDCA8, DLX5, URLC11 or NPTX1. The cell is further contacted with a transfection-enhancing agent. The cell is provided *in vitro, in vivo* or *ex vivo*. The subject is a mammal, *e*.*g*., a human, non-human primate, mouse, rat, dog, cat, horse, or cow. The cell is a lung cell. Alternatively, the cell is a tumor cell (*i*.*e*., cancer cell) such as a non-small cell cancer cell. For example, the cell is a non-small cell lung cancer cell. By the term "inhibiting cell growth" is meant that the treated cell proliferates at a lower rate or has decreased viability than an untreated cell. Cell growth is measured by proliferation assays known in the art.

By the term "siRNA," is meant a double stranded RNA molecule which prevents translation of a target mRNA. Standard techniques of introducing siRNA into the cell are used, including those in which DNA is a template from which RNA is transcribed. The siRNA includes a sense PKP3, CDCA1, CDCA8, DLX5, URLC11 or NPTX1 nucleic acid sequence, an anti-sense PKP3, CDCA1, CDCA8, DLX5, URIC11 or NPTX1 nucleic acid sequence or both. The siRNA may comprise two complementary molecules or may be constructed such that a single transcript has both the sense and complementary antisense sequences from the target gene, *e*.*g*., a hairpin, which, in some embodiments, leads to production of micro RNA (miRNA).

The method is used to alter gene expression in a cell in which expression of PKP3, CDCA1, CDCA8, DLX5, URIC11 or NPTX1 is up-regulated, *e*.*g*., as a result of malignant transformation of the cells. Binding of the siRNA to an PKP3, CDCA1, CDCA8, DLX5, URLC11 or NPTX1 transcript in the target cell results in a reduction in PKP3, CDCA1, CDCA8, DLX5, URLC11 or NPTX1 production by the cell. The length of the oligonucleotide is at least about 10 nucleotides and may be as long as the naturally-occurring PKP3, CDCA1, CDCA8, DLX5, URIC11 or NPTX1 transcript. Preferably, the oligonucleotide is about 19 to about 25 nucleotides in length. Most preferably, the oligonucleotide is less than about 100, about 75, about 50, or about 25 nucleotides in length. Examples of siRNA oligonucleotides of PKP3, CDCA1, CDCA8, DLX5, URLC11 or NPTX1 which inhibit PKP3, CDCA1, CDCA8, DLX5, URLC11 or NPTX1 expression in mammalian cells include oligonucleotides containing target sequences, for example, nucleotides of SEQ ID NOs: 58, 59, 60, 61, 62, 63, 64, 65, 66, 67, 68, 69, 70, 71 or 72.

Methods for designing double stranded RNA having the ability to inhibit gene expression in a target cell are known. (See for example, US Patent No. 6,506,559, herein incorporated by reference in its entirety). For example, a computer program for designing siRNAs is available from the Ambion website
(http://www.ambion.com/techlib/misc/siRNA_finder.html).
The computer program selects nucleotide sequences for siRNA synthesis based on the following protocol.

### Selection of siRNA Target Sites

1. Beginning with the AUG start codon of the transcript, scan downstream for AA dinucleotide sequences. Record the occurrence of each AA and the 3' adjacent 19 nucleotides as potential siRNA target sites. Tuschl et al. Targeted mRNA degradation by double-stranded RNA in vitro. Genes Dev 13(24): 3191-7 (1999), don't recommend designing siRNA to the 5' and 3' untranslated regions (UTRs) and regions near the start codon (within 75bases) as these may be richer in regulatory protein binding sites. UTR-binding proteins and/or translation initiation complexes may interfere with binding of the siRNA endonuclease complex.
2. Compare the potential target sites to the appropriate genome database (human, mouse, rat, etc.) and eliminate from consideration any target sequences with significant homology to other coding sequences. It is suggested to use BLAST, which can be found on the NCBI server at: www.ncbi.nlm.nih.gov/BLAST/.
3. Select qualifying target sequences for synthesis. Selecting several target sequences along the length of the gene to evaluate is typical.

Also included in the invention are isolated nucleic acid molecules that include the nucleic acid sequence of target sequences, for example, nucleotides of SEQ ID NOs: 58, 59, 60, 61, 62, 63, 64, 65, 66, 67, 68, 69, 70, 71 or 72, or a nucleic acid molecule that is complementary to the nucleic acid sequence of nucleotides of SEQ ID NOs: 58, 59, 60, 61, 62, 63, 64, 65, 66, 67, 68, 69, 70, 71 or 72. As used herein, an "isolated nucleic acid" is a nucleic acid removed from its original environment (e.g., the natural environment if naturally occurring) and thus, synthetically altered from its natural state. In the present invention, isolated nucleic acid includes DNA, RNA, and derivatives thereof. When the isolated nucleic acid is RNA or derivatives thereof, base "t" should be replaced with "u" in the nucleotide sequences. As used herein, the term "complementary"' refers to Watson-Crick or Hoogsteen base pairing between nucleotides units of a nucleic acid molecule, and the term "binding" means the physical or chemical interaction between two nucleic acids or compounds or associated nucleic acids or compounds or combinations thereof. Complementary nucleic acid sequences hybridize under appropriate conditions to form stable duplexes containing few or no mismatches. For the purposes of this invention, two sequences having 5 or fewer mismatches are considered to be complementary. Furthermore, the sense strand and antisense strand of the isolated nucleotide of the present invention, can form double stranded nucleotide or hairpin loop structure by the hybridization. In a preferred embodiment, such duplexes contain no more than 1 mismatch for every 10 matches. In an especially preferred embodiment, where the strands of the duplex are fully complementary, such duplexes contain no mismatches. The nucleic acid molecule is less than 2831, 2003, 2307, 1419, 2539 or 5071 nucleotides in length for PKP3, CDCA1, CDCA8, DLX5, URLC11 or NPTX1, respectively. For example, the nucleic acid molecule is less than about 500, about 200, or about 75 nucleotides in length. Also included in the invention is a vector containing one or more of the nucleic acids described herein, and a cell containing the vectors. The isolated nucleic acids of the present invention are useful for siRNA against PKP3, CDCA1, CDCA8, DLX5, URLC11 or NPTX1, or DNA encoding the siRNA. When the nucleic acids are used for siRNA or coding DNA thereof, the sense strand is preferably longer than about 19 nucleotides, and more preferably longer than about 21 nucleotides.

The invention is based in part on the discovery that the gene encoding PKP3, CDCA1, CDCA8, DLX5, URLC11 or NPTX1 is over-expressed in non-small cell lung cancer (NSCLC) compared to non-cancerous lung tissue. The cDNA of PKP3, CDCA1, CDCA8, DLX5, URLC11 or NPTX1 is 2831, 2003, 2307, 1419, 2539 or 5071 nucleotides in length, respectively. The nucleic acid sequences of PKP3, CDCA1, CDCA8, DLX5, URLC11 or NPTX1 are shown in SEQ ID NOs: 1, 3, 5, 7, 9 and 11. These nucleotide sequences comprise coding region in positions 75..2468 (NM_007183), 299..1693 (NM_145697), 114..956 (NM_018101), 195..1064 (BC006226), 134..1819 (NM_173514) and 139..1431 (NM_002522) which encode amino acid sequences of SEQ ID NOs: 2,4,6,8, 10 and 12, respectively. The sequence data are also available via following accession numbers.
PKP3: NM_007183
CDCA1: AI015982, NM_145697
CDCA8 (FLJ10468): NM_018101
DLX5: AI278397, BC006226
URLC11 (FLJ90709): AB105188, NM_173514
NPTX1: NM_002522

Transfection of siRNAs comprising SEQ ID NOs: 58, 59, 60, 61, 62, 63, 64, 65, 66, 67, 68, 69, 70, 71 or 72 resulted in a growth inhibition of NSCLC cell lines.

### Methods of inhibiting cell growth

The present invention relates to inhibiting cell growth, *i*.*e*, cancer cell growth by inhibiting expression of PKP3, CDCA1, CDCA8, DLX5, URLC11 or NPTX1. Expression of PKP3, CDCA1, CDCA8, DLX5, URLC11 or NPTX1 is inhibited, for example, by small interfering RNA (siRNA) that specifically target the PKP3, CDCA1, CDCA8, DLX5, URLC11 or NPTX1 gene. PKP3, CDCA1, CDCA8, DLX5, URLC11 or NPTX1 targets include, for example, nucleotides of SEQ ID NOs: 58, 59, 60, 61, 62, 63, 64, 65, 66, 67, 68, 69, 70, 71 or 72.

In non-mammalian cells, double-stranded RNA (dsRNA) has been shown to exert a strong and specific silencing effect on gene expression, which is referred as RNA interference (RNAi) (Sharp P A. RNAi and double-strand RNA. Genes Dev. 1 999 Jan 15; 13(2): 139-41.). dsRNA is processed into 20-23 nucleotides dsRNA called small interfering RNA (siRNA) by an enzyme containing RNase III motif. The siRNA specifically targets complementary mRNA with a multicomponent nuclease complex (Hammond SM et al. An RNA-directed nuclease mediates post-transcriptional gene silencing Drosophila cells.Nature. 2000 Mar 16; 404(6775): 293-6., Hannon GJ. RNA interference. Nature. 2002 Jul 11; 418(6894): 244-51.). In mammalian cells, siRNA composed of 20 or 21-mer dsRNA with 19 complementary nucleotides and 3' terminal noncomplementary dimmers of thymidine or uridine, have been shown to have a gene specific knock-down effect without inducing global changes in gene expression (Elbashir SM et al. Duplexes of 21-nucleotide RNAs mediate RNA interference in cultured mammalian cells. Nature. 2001 May 24; 411(6836): 494-8.). In addition, plasmids containing small nuclear RNA (snRNA) U6 or polymerase III HI-RNA promoter effectively produce such short RNA recruiting type III class of RNA polymerase III and thus can constitutively suppress its target mRNA (Miyagishi M et al. U6 promoter-driven siRNAs with four uridine 3' overhangs efficiently suppress targeted gene expression in mammalian cells. Nat Biotechnol. 2002 May; 20(5): 497-500., Brummelkamp TR et al. A System for Stable Expression of Short Interfering RNAs in Mammalian Cells. Science. Science. 296(5567); 550-553, April 19, 2002.).

The growth of cells is inhibited by contacting a cell with a composition containing a siRNA of PKP3, CDCA1, CDCA8, DLX5, URLC11 or NPTX1. The cell is further contacted with a transfection agent. Suitable transfection agents are known in the art. By the term "inhibition of cell growth" is meant that the cell proliferates at a lower rate or has decreased viability compared to a cell not exposed to the composition. Cell growth is measured by methods known in the art such as, the MTT cell proliferation assay.

The siRNA of PKP3, CDCA1, CDCA8, DLX5, URLC11 or NPTX1 is directed to a single target of PKP3, CDCA1, CDCA8, DLX5, URLC11 or NPTX1 gene sequence. Alternatively, the siRNA is directed to multiple target of PKP3, CDCA1, CDCA8, DLX5, URLC11 or NPTX1 gene sequences. For example, the composition contains siRNA of PKP3, CDCA1, CDCA8, DLX5, URLC11 or NPTX1 directed to two, three, four, or five or more PKP3, CDCA1, CDCA8, DLX5, URLC11 or NPTX1 target sequences. By the term "PKP3, CDCA1, CDCA8, DLX5, URLC11 or NPTX1 target sequence" is meant a nucleotide sequence that is identical to a portion of the PKP3, CDCA1, CDCA8, DLX5, URLC11 or NPTX1 gene. The target sequence can include the 5' untranslated (UT) region, the open reading frame (ORF) or the 3' untranslated region of the human PKP3, CDCA1, CDCA8, DLX5, URLC11 or NPTX1 gene. Alternatively, the siRNA is a nucleic acid sequence complementary to an upstream or downstream modulator of PKP3, CDCA1, CDCA8, DLX5, URLC11 or NPTX1 gene expression. Examples of upstream and downstream modulators include, a transcription factor that binds the PKP3, CDCA1, CDCA8, DLX5, URLC11 or NPTX1 gene promoter, a kinase or phosphatase that interacts with the PKP3, CDCA1, CDCA8, DLX5, URLC11 or NPTX1 polypeptide, a PKP3, CDCA1, CDCA8, DLX5, URLC11 or NPTX1 promoter or enhance.

siRNA of PKP3, CDCA1, CDCA8, DLX5, URLC11 or NPTX1 which hybridize to target mRNA decrease or inhibit production of the PKP3, CDCA1, CDCA8, DLX5, URLC11 or NPTX1 polypeptide product encoded by the PKP3, CDCA1, CDCA8, DLX5, URLC11 or NPTX1 gene by associating with the normally single-stranded mRNA transcript, thereby interfering with translation and thus, expression of the protein. Thus, siRNA molecules of the invention can be defined by their ability to hybridize specifically to mRNA or cDNA from a PKP3, CDCA1, CDCA8, DLX5, URLC11 or NPTX1 gene. For the purposes of this invention the terms "hybridize" or "hybridize specifically" are used to refer the ability of two nucleic acid molecules to hybridize under "stringent hybridization conditions." The phrase "stringent hybridization conditions" refers to conditions under which a nucleic acid molecule will hybridize to its target sequence, typically in a complex mixture of nucleic acids, but not detectably to other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 50°C.

The siRNA of the invention is less than about 500, about 200, about 100, about 50, or about 25 nucleotides in length. Preferably the siRNA is about 19 to about 25 nucleotides in length. Exemplary nucleic acid sequence for the production of PKP3, CDCA1, CDCA8, DLX5, URLC11 or NPTX1 siRNA include the sequences of nucleotides of SEQ ID NOs: 58, 59, 60, 61, 62, 63, 64, 65, 66, 67, 68, 69, 70, 71 or 72 as the target sequence. Furthermore, in order to enhance the inhibition activity of the siRNA, nucleotide "u" can be added to 3'end of the antisense strand of the target sequence. The number of "u"s to be added is at least about 2, generally about 2 to about 10, preferably about 2 to about 5. The added "u"s form single strand at the 3'end of the antisense strand of the siRNA.

The cell is any cell that expresses or over-expresses PKP3, CDCA1, CDCA8, DLX5, URLC11 or NPTX1. The cell is a lung cell. Alternatively, the cell is a tumor cell such as a carcinoma, adenocarcinoma, blastoma, leukemia, myeloma, or sarcoma. The cell is a non-small cell lung cancer cell.

An siRNA of PKP3, CDCA1, CDCA8, DLX5, URLC11 or NPTX1 is directly introduced into the cells in a form that is capable of binding to the mRNA transcripts. Alternatively, the DNA encoding the siRNA of PKP3, CDCA1, CDCA8, DLX5, URLC11 or NPTX1 is in a vector.

Vectors are produced for example by cloning a PKP3, CDCA1, CDCA8, DLX5, URLC11 or NPTX1 target sequence into an expression vector operatively-linked regulatory sequences flanking the PKP3, CDCA1, CDCA8, DLX5, URLC11 or NPTX1 sequence in a manner that allows for expression (by transcription of the DNA molecule) of both strands (Lee, N.S., Dohjima, T., Bauer, G., Li, H., Li, M.-J., Ehsani, A.,Salvaterra, P., and Rossi, J. (2002) Expression of small interfering RNAs targeted against HIV-1 rev transcripts in human cells. Nature Biotechnology 20 : 500-505.). An RNA molecule that is antisense to PKP3, CDCA1, CDCA8, DLX5, URLC11 or NPTX1 mRNA is transcribed by a first promoter (*e*.*g*., a promoter sequence 3' of the cloned DNA) and an RNA molecule that is the sense strand for the PKP3, CDCA1, CDCA8, DLX5, URLC11 or NPTX1 mRNA is transcribed by a second promoter (*e*.*g*., a promoter sequence 5' of the cloned DNA). The sense and antisense strands hybridize *in vivo* to generate siRNA constructs for silencing of the PKP3, CDCA1, CDCA8, DLX5, URLC11 or NPTX1 gene. Alternatively, two constructs are utilized to create the sense and anti-sense strands of a siRNA construct. Cloned PKP3, CDCA1, CDCA8, DLX5, URLC11 or NPTX1 can encode a construct having secondary structure, e.g., hairpins, wherein a single transcript has both the sense and complementary antisense sequences from the target gene. A loop sequence consisting of an arbitrary nucleotide sequence can be located between the sense and antisense sequence in order to form the hairpin loop structure. Thus, the present invention also provides siRNA having the general formula 5'-[A]-[B]-[A']-3', wherein [A] is a ribonucleotide sequence corresponding to a sequence that specfically hybridizes to an mRNA or a cDNA from a PKP3, CDCA1, CDCA8, DLX5, URLC11 or NPTX1 gene. In preferred embodiments, [A] is a ribonucleotide sequence corresponding to a sequence selected from the group consisting of nucleotides of SEQ ID NOs: 58, 59, 60, 61, 62, 63, 64, 65, 66, 67, 68, 69, 70, 71 or 72,

[B] is a ribonucleotide sequence consisting of 3 to 23 nucleotides, and
[A'] is a ribonucleotide sequence consisting of the complementary sequence of [A]. The region [A] hybridizes to [A'], and then a loop consisting of region [B] is formed. The loop sequence may be preferably about 3 to about 23 nucleotides in length. The loop sequence, for example, can be selected from group consisting of following sequences (http://www.ambion.com/techlib/tb/tb_506.html). Furthermore, loop sequence consisting of 23 nucleotides also provides active siRNA (Jacque, J.-M., Triques, K., and Stevenson, M. (2002) Modulation of HIV-1 replication by RNA interference. Nature 418 : 435-438.).

CCC, CCACC or CCACACC: Jacque, J. M, Triques, K., and Stevenson, M (2002) Modulation of HIV-1 replication by RNA interference. Nature, Vol. 418: 435-438.

UUCG: Lee, N.S., Dohjima, T., Bauer, G., Li, H., Li, M.-J., Ehsani, A., Salvaterra, P., and Rossi, J. (2002) Expression of small interfering RNAs targeted against HIV-1 rev transcripts in human cells. Nature Biotechnology 20 : 500-505. Fruscoloni, P., Zamboni, M., and Tocchini-Valentini, G. P. (2003) Exonucleolytic degradation of double-stranded RNA by an activity in Xenopus laevis germinal vesicles. Proc. Natl. Acad. Sci. USA 100(4): 1639-1644.

UUCAAGAGA: Dykxhoorn, D. M., Novina, C. D., and Sharp, P. A. (2002) Killing the messenger: Short RNAs that silence gene expression. Nature Reviews Molecular Cell Biology 4: 457-467.

For example, preferable siRNAs having hairpin loop structure of the present invention are shown below. In the following structure, the loop sequence can be selected from group consisting of, CCC, UUCG, CCACC, CCACACC, and UUCAAGAGA. Preferable loop sequence is UUCAAGAGA ("ttcaagaga" in DNA).
ccuguggcaguacaacaag-[b]-cuuguuguacugccacagg(for target sequence of SEQ ID NO:58)
ugccagacaagaaguggug-[b]-caccacuucuugucuggca(for target sequence of SEQ ID NO:59)
gaugcugcugaaagggaga-[b]-ucucccuuucagcagcauc(for target sequence of SEQ ID NO:60)
cagcagaagcuauucagac-[b]-gucugaauagcuucugcug(for target sequence of SEQ ID NO:61)
gguguccuccauccaagaa-[b]-uucuuggauggaggacacc(for target sequence of SEQ ID NO:62)
gccgugcuaacacuguuac-[b]-guaacaguguuagcacggc(for target sequence of SEQ ID NO:63)
gaagcucuccaaccgucuc-[b]-gagacgguuggagagcuuc(for target sequence of SEQ ID NO:64)
gacucaguaccucgccuug-[b]-caaggcgagguacugaguc(for target sequence of SEQ ID NO:65)
gguuucagaagacucagua-[b]-uacugagucuucugaaacc(for target sequence of SEQ ID NO:66)
gugcagccagcucaaucaa-[b]-uugauugagcuggcugcac(for target sequence of SEQ ID NO:67) gagaauucauuacuacagc-[b]-gcuguaguaaugaauucuc(for target sequence of SEQ ID NO:68) ggauauuccugcuguucca-[b]-uggaacagcaggaauaucc(for target sequence of SEQ ID NO:69) gauauucaggagcagcaug-[b]-caugcugcuccugaauauc(for target sequence of SEQ ID NO:70) ggagaccauccugagccag-[b]-cuggcucaggauggucucc(for target sequence of SEQ ID NO:71) guggaccuucgaggccugu-[b]-acaggccucgaagguccac(for target sequence of SEQ ID NO:72)

The regulatory sequences flanking the PKP3, CDCA1, CDCA8, DLX5, URLC11 or NPTX1 sequence are identical or are different, such that their expression can be modulated independently, or in a temporal or spatial manner. siRNAs are transcribed intracellularly by cloning the PKP3, CDCA1, CDCA8, DLX5, URLC11 or NPTX1 gene templates into a vector containing, *e*.*g*., a RNA polymerase III transcription unit from the small nuclear RNA (snRNA) U6 or the human H1 RNA promoter. For introducing the vector into the cell, transfection-enhancing agent can be used. FuGENE (Roche Diagnostices), Lipofectamine 2000 (Invitrogen), Oligofectamine (Invitrogen), and Nucleofector (Wako pure Chemical) are useful as the transfection-enhancing agent.

Oligonucleotides and oligonucleotides complementary to various portions of PKP3, CDCA1, CDCA8, DLX5, URLC 11 or NPTX1 mRNA were tested *in vitro* for their ability to decrease production of PKP3, CDCA1, CDCA8, DLX5, URLC11 or NPTX1 in tumor cells (e.g., using the NSCLC cell line such as A549, LC319 or LC176.) according to standard methods. A reduction in PKP3, CDCA1, CDCA8, DLX5, URLC11 or NPTX1 gene product in cells contacted with the candidate siRNA composition compared to cells cultured in the absence of the candidate composition is detected using specific antibodies of PKP3, CDCA1, CDCA8, DLX5, URLC11 or NPTX1 or other detection strategies. Sequences which decrease production of PKP3, CDCA1, CDCA8, DLX5, URLC11 or NPTX1 in *in vitro* cell-based or cell-free assays are then tested for there inhibitory effects on cell growth. Sequences which inhibit cell growth *in vitro* cell-based assay are test *in vivo* in rats or mice to confirm decreased PKP3, CDCA1, CDCA8, DLX5, URLC11 or NPTX1 production and decreased tumor cell growth in animals with malignant neoplasms.

### Methods of treating malignant tumors

Patients with tumors characterized as over-expressing PKP3, CDCA1, CDCA8, DLX5, URLC11 or NPTX1 are treated by administering siRNA of PKP3, CDCA1, CDCA8, DLX5, URLC11 or NPTX1. siRNA therapy is used to inhibit expression of PKP3, CDCA1, CDCA8, DLX5, URLC11 or NPTX1 in patients suffering from or at risk of developing, for example, non-small cell lung cancer cell. Such patients are identified by standard methods of the particular tumor type. Non-small cell lung cancer cell is diagnosed for example, computer tomography, thoracoscopy, chest X-ray, sputum cytology, TBB(trans-bronchial biopsy), MRI (Magnetic Resonance Imaging) or FDG-PET (¹⁸F-fluorodeoxyglucose-positron emission tomography).

Treatment is efficacious if the treatment leads to clinical benefit such as, a reduction in expression of PKP3, CDCA1, CDCA8, DLX5, URLC11 or NPTX1, or a decrease in size, prevalence, or metastatic potential of the tumor in the subject. When treatment is applied prophylactically, "efficacious" means that the treatment retards or prevents tumors from forming or prevents or alleviates a symptom of clinical symptom of the tumor. Efficaciousness is determined in association with any known method for diagnosing or treating the particular tumor type.

siRNA therapy is carried out by administering to a patient a siRNA by standard vectors encoding the siRNAs of the invention and/or gene delivery systems such as by delivering the synthetic siRNA molecules. Typically, synthetic siRNA molecules are chemically stabilized to prevent nuclease degradation *in vivo*. Methods for preparing chemically stabilized RNA molecules are well known in the art. Typically, such molecules comprise modified backbones and nucleotides to prevent the action of ribonucleases. Other modifications are also possible, for example, cholesterol-conjugated siRNAs have shown improved pharmacological properties.(Song et al. Nature Med. 9:347-351 (2003)): Suitable gene delivery systems may include liposomes, receptor-mediated delivery systems, or viral vectors such as herpes viruses, retroviruses, adenoviruses and adeno-associated viruses, among others. A therapeutic nucleic acid composition is formulated in a pharmaceutically acceptable carrier. The therapeutic composition may also include a gene delivery system as described above. Pharmaceutically acceptable carriers are biologically compatible vehicles which are suitable for administration to an animal, *e*.*g*., physiological saline. A therapeutically effective amount of a compound is an amount which is capable of producing a medically desirable result such as reduced production of a PKP3, CDCA1, CDCA8, DLX5, URLC11 or NPTX1 gene product, reduction of cell growth, *e*.*g*., proliferation, or a reduction in tumor growth in a treated animal.

Parenteral administration, such as intravenous, subcutaneous, intramuscular, and intraperitoneal delivery routes, may be used to deliver siRNA compositions of PKP3, CDCA1, CDCA8, DLX5, URLC11 or NPTX1. For treatment of pulmonary tumors, airway administration, injection under CT guidance or with thoracoscopy is useful.

Dosages for any one patient depends upon many factors, including the patient's size, body surface area, age, the particular nucleic acid to be administered, sex, time and route of administration, general health, and other drugs being administered concurrently. Dosage for intravenous administration of nucleic acids is from approximately 10⁶ to 10²² copies of the nucleic acid molecule.

The polynucleotides are administered by standard methods, such as by injection into the interstitial space of tissues such as muscles or skin, introduction into the circulation or into body cavities or by inhalation or insufflation. Polynucleotides are injected or otherwise delivered to the animal with a pharmaceutically acceptable liquid carrier, *e.g.,* a liquid carrier, which is aqueous or partly aqueous. The polynucleotides are associated with a liposome (*e*.*g*., a cationic or anionic liposome). The polynucleotide includes genetic information necessary for expression by a target cell, such as a promoter.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

### Brief Description of the Drawings

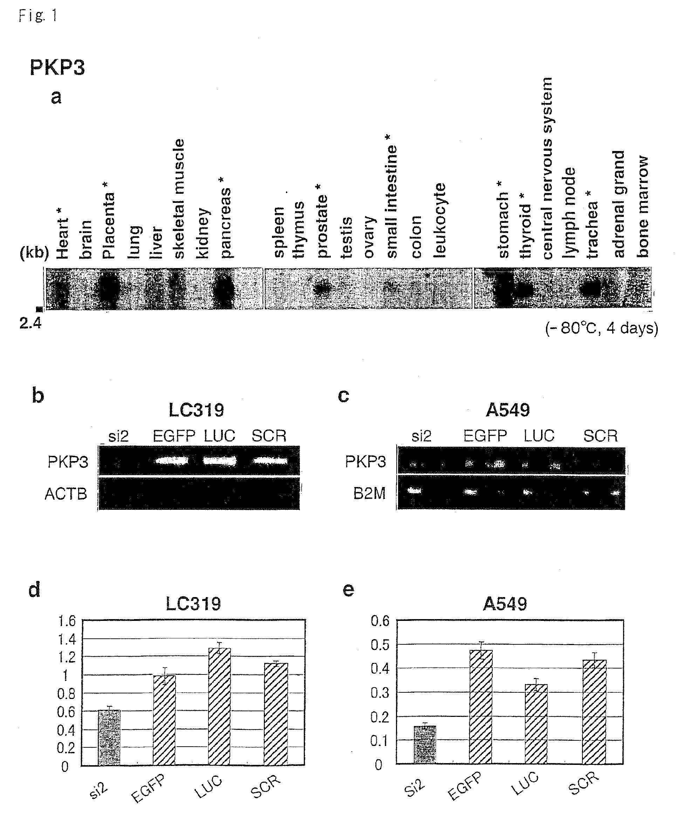

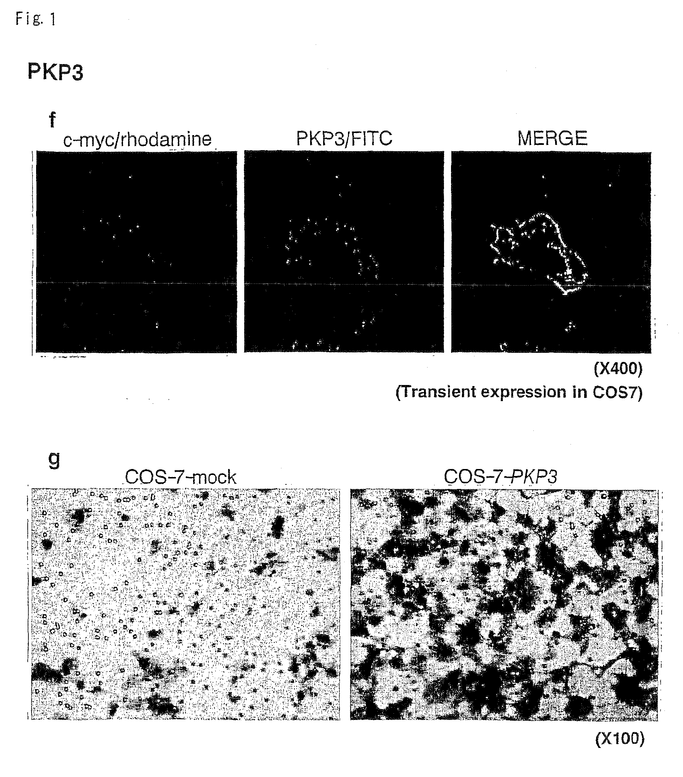

Fig. 1 depicts characterization of PKP3. Fig. 1a shows a photograph of a Northern-blot illustrating *PKP3* expression in normal tissue samples. Fig. 1b and 1c show a photograph showing inhibition of *PKP3* mRNA by siRNA for *PKP3.* Fig. 1d and 1e show a bar chart(MTT assay) showing inhibition of cell growth by siRNA for *PKP3.* In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble. Fig. 1f is a photograph showing subcellular localization of PKP3 by immunocytochemical analysis, when the COS-7 cells were transfected with the c-myc-His tagged PKP3 expression plasmid. (left panel): PKP3 detected with anti-c-myc-rhodamine antibody, (middle): PKP3 detected with anti-PKP3 antibody conjugated to FITC, (right): MERGE image including DAPI stain. PKP3/c-myc-His protein was mainly detected in perinucleus and cytoplasmic membrane. Fig. 1g shows Matrigel invasion assay demonstrating the promotion of COS-7 cell invasive nature in Matrigel matrix when the human PKP3 expression plasmids were transfected (Giemsa staining).

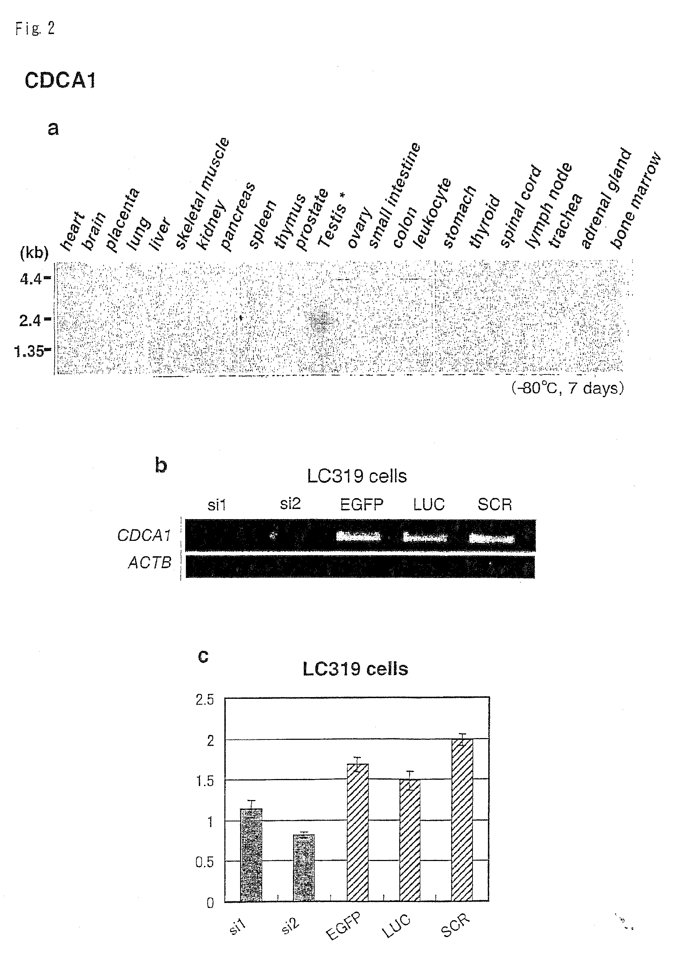

Fig. 2 depicts characterization of CDCA1. Fig. 2a shows a photograph of a Northern-blot illustrating *CDCA1* expression in normal tissue samples: Fig. 2b shows a photograph showing inhibition of *CDCA1* mRNA by siRNA for *CDCA1.* Fig. 2c is a bar chart (MTT assay) showing inhibition of cell growth by siRNA for *CDCA1.* In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble.

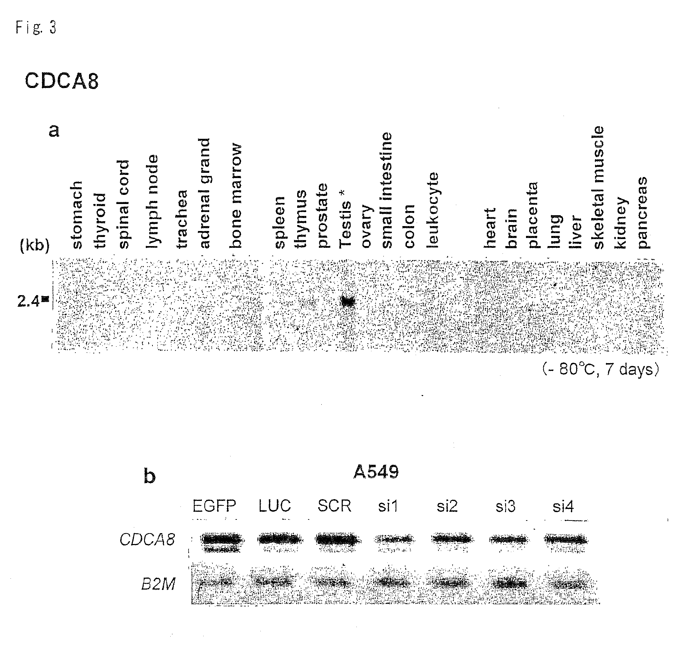

Fig. 3 depicts characterization of CDCA8. Fig. 3a shows a photograph of a Northern-blot illustrating *CDCA8* expression in normal tissue samples. Fig. 3b shows a photograph showing inhibition of *CDCA8* mRNA by siRNA for *CDCA8.* In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble.

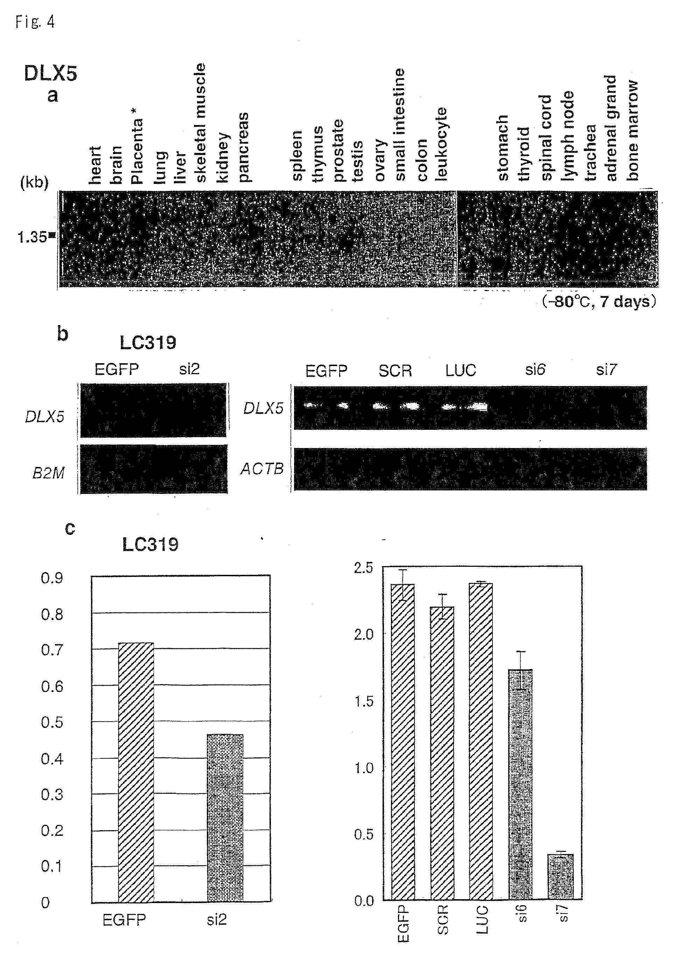

Fig. 4 depicts characterization of DLX5. Fig. 4a shows a photograph of a Northern-blot illustrating *DLX5* expression in normal tissue samples. Fig. 4b shows a photograph showing inhibition of DLX5 mRNA by siRNA for DLX5. Fig. 4c is a bar chart (MTT assay) showing inhibition of cell growth by siRNAfor *DLX5.* In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble.

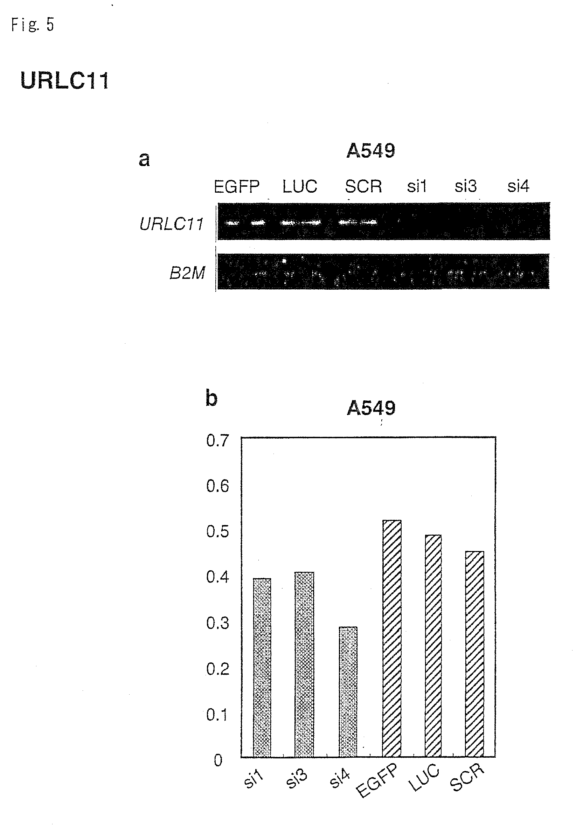

Fig. 5 depicts characterization of URLC11. Fig. 5a shows a photograph showing inhibition of URLC11 mRNA by siRNA for URLC11. Fig. 5b is a bar chart (MTT assay) showing inhibition of cell growth by siRNA for URLC 11. In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble.

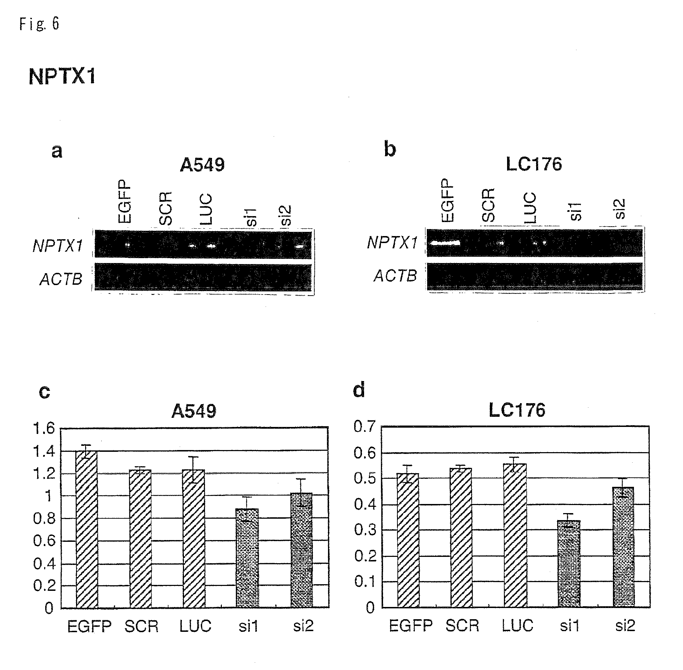

Fig. 6 depicts characterization of NPTXL. Fig. 6a and 6b show a photograph showing inhibition of *NPTX1* mRNA by siRNA for *NPTX1*. Fig. 6c and 6d are bar charts (MTT assay) showing inhibition of cell growth by siRNA for *NPTX1.* In control siRNA, (EGFP) shows NSCLC cells transfected with siRNA for EGFP; (LUC), NSCLC cells transfected with siRNA for Luciferase-GL2; and (SCR), NSCLC cells transfected with siRNA for Scramble.

### Best Mode for Carrying out the Invention

The invention will be further described in the following examples, which do not limit the scope of the invention described in the claims.

### [Example 1] General Methods

### Cell lines and tissue specimens

Twenty-one human NSCLC cell lines were grown in monolayers in appropriate medium supplemented with 5 or 10% fetal bovine serum (see Table 1).

**Table 1**

| Cell line name | | Medium |
|---|---|---|
| **adenocarcinoma (ADC)** | | |
| | A549 | RPMI-1640(10%FBS) |
| | NCI-H23 | RPMI-1640(10%FBS) |
| | NCI-H522 | RPMI-1640(10%FBS) |
| | LC174 | RPMI-1640(10%FBS) |
| | LC176 | RPMI-1640(10%FBS) |
| | LC319 | RPMI-1640(10%FBS) |
| | PC-3 | DMEM(10%FBS) |
| | PC-9 | DMEM(10%FBS) |
| | PC14 | RPMI-1640(10%FBS) |
| | PC14-PE6 | RPMI-1640(10%FBS) |
| | NCI-H1373 | RPMI-1640(10%FBS) |
| | NCI-H1435 | F12+DMEM(5%FBS)+EGF(+) |
| | NCI-H1793 | F12+DMEM(5%FBS)+Glu |
| | SK-LU-1 | DMEM(10%FBS) |
| BAC | NCI-H358 | RPMI-1640(10%FBS) |
| BAC | NCI-H1650 | RPMI-1640(10%FBS) |
| BAC | SW1573 | Leibovitz's L-15(10%FBS) |
| **squamous cell carcinoma (SCC)** | | |
| | RERF-LC-AI | DMEM(IU%FBS) |
| | SW-900 | Leibovitz's L-15(10%FBS) |
| | SK-MES-1 | DMEM(10%FBS) |
| adenosquamous | NCI-H596 | RPMI-1640(10%FBS) |

### Isolation of over-expressing genes in NSCLC cells by using cDNA microarray

Fabrication of the cDNA microarray slides has been described (Ono K, Tanaka T, Tsunoda T, Kitahara O, Kihara C, Okamoto A, Ochiai K, Takagi T, and Nakamura Y. Cancer-Res., 60: 5007-5011, 2000). For each analysis of expression profiles it was prepared duplicate sets of cDNA microarray slides containing approximately 23,040 DNA spots, to reduce experimental fluctuation. Briefly, total RNA was purified from NSCLC cell. T7-based RNA amplification was carried out to obtain adequate RNA for microarray experiments. As a control probe, normal human lung poly[A] RNA (CLONTEC) was amplified in the same way. Aliquots of amplified RNA from NSCLC cell and normal lung cell were labeled by reverse transcription with Cy5-dCTP and Cy3-dCTP, respectively (Amersham Biosciences). Hybridization, washing, and detection were carried out as described previously (Ono K, Tanaka T, Tsunoda T, Kitahara O, Kihara C, Okamoto A, Ochiai K, Takagi T, and Nakamura Y. Cancer Res., 60: 5007-5011, 2000). Subsequently, among the up-regulated genes, it was focused six genes, PKP3, CDCA1, CDCA8, DLX5, URLC11 and NPTX1.

### RNA preparation and RT-PCR

Total RNA was extracted from cultured cells and clinical tL ssues using Trizol reagent (Life Technologies, Inc., Gaithersburg, MD, USA) according to the manufacturer's protocol. Extracted RNA was treated with DNase I (Roche Diagnostics, Basel, Switzerland) and reverse transcribed to single-stranded cDNAs using oligo(dT)₁₂₋₁₈ primer with Superscript II reverse transcriptase (Life Technologies, Inc.). Appropriate dilutions of each single-stranded cDNA for subsequent PCR amplification were prepared by monitoring the beta-actin (*ACTB*) or beta-2-microglobulin gene (*B2M*) as the quantitative controls. All reactions involved initial denaturation at 94 °C for 2 min, followed by 18 (for *ACTB* or *B2M*) or 25-30 cycles (for each gene of the present invention) of 94 °C for 30s, 58-62 °C for 30s and 72 °C for 45s on GeneAmp PCR system 9700 (Applied Biosystems). The primer sequences are listed below:
For PKP3
   Forward; 5'-ATGCAGGACGGTAACTTCCTGC-3' (SEQ ID NO: 73)
   Reverse; 5'-TGGGCCCAGGAAGTCCTCCTT-3' (SEQ ID NO: 74)
For CDCA1
   Forward; 5'-GAGAAACTGAAGTCCCAGGAAAT-3' (SEQ ID NO: 75)
   Reverse; 5'-CTGATACTTCCATTCGCTTCAAC-3' (SEQ ID NO: 76)
For CDCA8
   Forward; 5'- CATCTGGCATTTCTGCTCTCTAT -3'(SEQ ID NO: 77)
   Reverse; 5'-CTCAGGGAAAGGAGAATAAAAGAAC-3' (SEQ ID NO: 78)
For DLX5
   Forward; 5'-GGGACTACTGTGTTTTGCTGTTC-3' (SEQ ID NO: 79)
   Reverse; 5'-TGAGGTCATAGATTTCAAGGCAC-3' (SEQ ID NO: 80)
For URLC11
   Forward; 5'- CTAGGAAGAAATCATGCTGGGTT -3' (SEQ ID NO: 81)
   Reverse; 5'- GTATTTGGCTTACTGTCCCAAAC -3' (SEQ ID NO: 82)
For NPTX1
   Forward; 5'-TAACCTTGATAGAAGAACCTTGG-3' (SEQ ID NO: 83)
   Reverse; 5'-GCAAATGAGACAAAATTGGGAC-3' (SEQ ID NO: 84)

### Northern blot analysis

³²P-labeled PCR products were hybridized with human multiple-tissue blots (BD Biosciences Clontech). Prehybridization, hybridization and washing were performed according to the supplier's recommendations. The blots were autoradiographed on intensifying screens at -80°C for 96-168 hours.

To determine tissue distribution and the size of each of the genes, human multiple tissue Northern blot analysis was performed using human cDNA as a probe.

### Construction of psiH1BX3. 0 Plasmid

To prepare plasmid vector expressing short interfering RNA (siRNA), the genomic fragment of H1RNA gene containing its promoter region was amplified by PCR using a set of primers, 5'-TGGTAGCCAAGTGCAGGTTATA-3' (SEQ ID NO; 85), and 5'- CCAAAGGGTTTCTGCAGTTTCA-3' (SEQ ID NO; 86) and human placental DNA as a template. The product was purified and cloned into pCR2.0 plasmid vector using a TA cloning kit according to the supplier's protocol (Invitrogen). The *Bam*HI and *Xho*I fragment containing *HIRNA* was cloned into pcDNA3.1(+)nucleotides 1257 to 56, and the fragment was amplified by PCR using
5'-TGCGGATCCAGAGCAGATTGTACTGAGAGT-3' (SEQ ID NO; 87) and
5'- CTCTATCTCGAGTGAGGCGGAAAGAACCA-3' (SEQ ID NO; 88).

The ligated DNA became the template for PCR amplification with primers,
5'- TTTAAGCTTGAAGACCATTTTTGGAAAAAAAAAAAAAAAAAAAAAAAC-3' (SEQ ID NO; 89) and
5'-TTTAAGCTTGAAGACATGGGAAAGAGTGGTCTCA-3' (SEQ ID NO; 90).

The product was digested with *Hin*dIII, and subsequently self-ligated to produce psiH1BX3. 0 vector plasmid having a nucleotide sequence shown in SEQ ID NO: 91.

The DNA flagment encoding siRNA was inserted into the GAP at nucleotide 489-492 as indi cated (-) in the following plasmid sequence (SEQ ID NO: 91). 
GACGGATCGGGAGATCTCCCGATCCCCTATGGTGCACTCTCAGTACAATCTGCTCTGGATCC
ACTAGTAACGGCCGCCAGTGTGCTGGAATTCGGCTTGGTAGCCAAGTGCAGGTTATAGGGA
GCTGAAGGGAAGGGGGTCACAGTAGGTGGCATCGTTCCTTTCTGACTGCCCGCCCCCCGCA
TGCCGTCCCGCGATATTGAGCTCCGAACCTCTCGCCCTGCCGCCGCCGGTGCTCCGTCGCCG
CCGCGCCGCCATGGAATTCGAACGCTGACGTCATCAACCCGCTCCAAGGAATCGCGGGCCC
AGTGTCACTAGGCGGGAACACCCAGCGCGTGCGCCCTGGCAGGAAGATGGCTGTGAGG
GACAGGGGAGTGGCGCCCTGCAATATTTGCATGTCGCTATGTGTTCTGGGAAATCACCATAA
 
ACGTGAAATGTCTTTGGATTTGGGAATCTTATAAGTTCTGTATGAGACCACTCTTTCCC- - - -T
TTTTGGGAAAAAAAAAAAAAAAAAAAAACGAAACCGGGCCGGGCGCGGTGGTTCACGC
CTATAATCCCAGCACTTTGGGAGGCCGAGGCGGGCGGATCACAAGGTCAGGAGGTCGAGAC
CATCCAGGCTAACACGGTGAAACCCCCCCCATCTCTACTAAAAAAAAAAAATACAAAAAA
TTAGCCATTAGCCGGGCGTGGTGGCGGGCGCCTATAATCCCAGCTACTTGGGAGGCTGAAGC
AGAATGGCGTGAACCCGGGAGGCGGACGTTGCAGTGAGCCGAGATCGCGCCGACTGCATTC
CAGCCTGGGCGACAGAGCGAGTCTCAAAAAAAAAACCGAGTGGAATGTGAAAAGCTCCGT
GAAACTGCAGAAACCCAAGCCGAATTCTGCAGATATCCATCACACTGGCGGCCGCTCGAGT
GAGGCGGAAAGAACCAGCTGGGGCTCTAGGGGGTATCCCCACGCGCCCTGTAGCGGCGCAT
TAAGCGCGGCGGGTGTGGTGGTTACGCGCAGCGTGACCGCTACACTTGCCAGCGCCCTAGC
GCCCGCTCCTTTCGCTTTCTTCCCTTCCTTTCTCGCCACGTTCGCCGGCTTTCCCCGTCAAGC
TCTAAATCGGGGGCTCCCTTTAGGGTTCCGATTTAGTGCTTTACGGCACCTCGACCCCAAAA
AACTTGATTAGGGTGATGGTTCACGTAGTGGGCCATCGCCCTGATAGACGGTTTTTCGCCCTT
TGACGTTGGAGTCCACGTTCTTTAATAGTGGACTCTTGTTCCAAACTGGAACAACACTCAAC
CCTATCTCGGTCTATTCTTTTGATTTATAAGGGATTTTGCCGATTTCGGCCTATTGGTTAAAAA
ATGAGCTGATTTAACAAAAATTTAACGCGAATTAATTCTGTGGAATGTGTGTCAGTTAGGGTG
TGGAAAGTCCCCAGGCTCCCCAGCAGGCAGAAGTATGCAAAGCATGCATCTCAATTAGTCA
GCAACCAGGTGTGGAAAGTCCCCAGGCTCCCCAGCAGGCAGAAGTATGCAAAGCATGCATC
TCAATTAGTCAGCAACCATAGTCCCGCCCCTAACTCCGCCCATCCCGCCCCTAACTCCGCCC
AGTTCCGCCCATTCTCCGCCCCATGGCTGACTAATTTTTTTTATTTATGCAGAGGCCGAGGCC
GCCTCTGCCTCTGAGCTATTCCAGAAGTAGTGAGGAGGCTTTTTTGGAGGCCTAGGCTTTTG
CAAAAAGCTCCCGGGAGCTTGTATATCCATTTTCGGATCTGATCAAGAGACAGGATGAGGAT
CGTTTCGCATGATTGAACAAGATGGATTGCACGCAGGTTCTCCGGCCGCTTGGGTGGAGAG
GCTATTCGGCTATGACTGGGCACAACAGACAATCGGCTGCTCTGATGCCGCCGTGTTCCGGC
TGTCAGCGCAGGGGCGCCCGGTTCTTTTTGTCAAGACCGACCTGTCCGGTGCCCTGAATGA
ACTGCAGGACGAGGCAGCGCGGCTATCGTGGCTGGCCACGACGGGCGTTCCTTGCGCAGCT
GTGCTCGACGTTGTCACTGAAGCGGGAAGGGACTGGCTGCTATTGGGCGAAGTGCCGGGGC
AGGATCTCCTGTCATCTCACCTTGCTCCTGCCGAGAAAGTATCCATCATGGCTGATGCAATGC
GGCGGCTGCATACGCTTGATCCGGCTACCTGCCCATTCGACCACCAAGCGAAACATCGCATC
GAGCGAGCACGTACTCGGATGGAAGCCGGTCTTGTCGATCAGGATGATCTGGACGAAGAGC
ATCAGGGGCTCGCGCCAGCCGAACTGTTCGCCAGGCTCAAGGCGCGCATGCCCGACGGCGA
GGATCTCGTCGTGACCCATGGCGATGCCTGCTTGCCGAATATCATGGTGGAAAATGGCCGCT
TTTCTGGATTCATCGACTGTGGCCGGCTGGGTGTGGCGGACCGCTATCAGGACATAGCGTTG
GCTACCCGTGATATTGCTGAAGAGCTTGGCGGCGAATGGGCTGACCGCTTCCTCGTGCTTTA
CGGTATCGCCGCTCCCGATTCGCAGCGCATCGCCTTCTATCGCCTTCTTGACGAGTTCTTCTG
 
AGCGGGACTCTGGGGTTCGAAATGACCGACCAAGCGACGCCCAACCTGCCATCACGAGATT
TCGATTCCACCGCCGCCTTCTATGAAAGGTTGGGCTTCGGAATCGTTTTCCGGGACGCCGGC
TGGATGATCCTCCAGCGCGGGGATCTCATGCTGGAGTTCTTCGCCCACCCCAACTTGTTTATT
GCAGCTTATAATGGTTACAAATAAAGCAATAGCATCACAAATTTCACAAATAAAGCATTTTTT
TCACTGCATTCTAGTTGTGGTTTGTCCAAACTCATCAATGTATCTTATCATGTCTGTATACCGT
CGACCTCTAGCTAGAGCTTGGCGTAATCATGGTCATAGCTGTTTCCTGTGTGAAATTGTTATC
CGCTCACAATTCCACACAACATACGAGCCGGAAGCATAAAGTGTAAAGCCTGGGGTGCCTA
ATGAGTGAGCTAACTCACATTAATTGCGTTGCGCTCACTGCCCGCTTTCCAGTCGGGAAACC
TGTCGTGCCAGCTGCATTAATGAATCGGCCAACGCGCGGGGAGAGGCGGTTTGCGTATTGG
GCGCTCTTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCAGGAAAG
AACATGTGAGCAAAAGGCCAGCAAAAGGCCAGGAACCGTAAAAAGGCCGCGTTGCTGGCG
TTTTTCCATAGGCTCCGCCCCCCTGACGAGCATCACAAAAATCGACGCTCAAGTCAGAGGTG
GCGAAACCCGACAGGACTATAAAGATACCAGGCGTTTCCCCCTGGAAGCTCCCTCGTGCGC
TCTCCTGTTCCGACCCTGCCGCTTACCGGATACCTGTCCGCCTTTCTCCCTTCGGGAAGCGTG
GCGCTTTCTCATAGCTCACGCTGTAGGTATCTCAGTTCGGTGTAGGTCGTTCGCTCCAAGCTG
GGCTGTGTGCACGAACCCCCCGTTCAGCCCGACCGCTGCGCCTTATCCGGTAACTATCGTCT
TGAGTCCAACCCGGTAAGACACGACTTATCGCCACTGGCAGCAGCCACTGGTAACAGGATT
AGCAGAGCGAGGTATGTAGGCGGTGCTACAGAGTTCTTGAAGTGGTGGCCTAACTACGGCT
ACACTAGAAGAACAGTATTTGGTATCTGCGCTCTGCTGAAGCCAGTTACCTTCGGAAAAAGA
GTTGGTAGCTCTTGATCCGGCAAACAAACCACCGCTGGTAGCGGTTTTTTTGTTTGCAAGCA
GCAGATTACGCGCAGAAAAAAAGGATCTCAAGAAGATCCTTTGATCTTTTCTACGGGGTCTG
ACGCTCAGTGGAACGAAAACTCACGTTAAGGGATTTTGGTCATGAGATTATCAAAAAGGATC
TTCACCTAGATCCTTTTAAATTAAAAATGAAGTTTTAAATCAATCTAAAGTATATATGAGTAAA
CTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCAT
CTGGCCCCAGTGCTGCAATGATACCGCGAGACCCACGCTCACCGGCTCCAGATTTATCAGCA
ATAAACCAGCCAGCCGGAAGGGCCGAGCGCAGAAGTGGTCCTGCAACTTTATCCGCCTCCA
TCCAGTCTATTAATTGTTGCCGGGAAGCTAGAGTAAGTAGTTCGCCAGTTAATAGTTTGCGCA
ACGTTGTTGCCATTGCTACAGGCATCGTGGTGTCACGCTCGTCGTTTGGTATGGCTTCATTCA
GCTCCGGTTCCCAACGATCAAGGCGAGTTACATGATCCCCCATGTTGTGCAAAAAAGCGGTT
AGCTCCTTCGGTCCTCCGATCGTTGTCAGAAGTAAGTTGGCCGCAGTGTTATCACTCATGGTT
ATGGCAGCACTGCATAATTCTCTTACTGTCATGCCATCCGTAAGATGCTTTTCTGTGACTGGT
GAGTACTCAACCAAGTCATTCTGAGAATAGTGTATGCGGCGACCGAGTTGCTCTTGCCCGGC
GTCAATACGGGATAATACCGCGCCACATAGCAGAACTTTAAAAGTGCTCATCATTGGAAAAC
 
GTTCTTCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAGCAAA
AACAGGAAGGCAAAATGCCGCAAAAAAGGGAATAAGGGCGACACGGAAATGTTGAATACT
CATACTCTTCCTTTTTCAATATTATTGAAGCATTTATCAGGGTTATTGTCTCATGAGCGGATACA
TATTTGAATGTATTTAGAAAAATAAACAAATAGGGGTTCCGCGCACATTTCCCCGAAAAGTG
CCACCTGACGTC
 siRNA expression vectors were prepared by cloning the double-stranded oligonucleotide into the Bbs1 site of the psiH1BX vector.

### siRNA-expressing constructs

The nucleotide sequence of the siRNAs were designed using an siRNA design computer program available from the Ambion website
(http://www.ambion.com/techlib/misc/siRNA_finder.html). Briefly, nucleotide sequences for siRNA synthesis are selected using the following protocol.

### Selection of siRNA Target Sites:

1. Starting with the AUG start codon of the each gene transcript, scan downstream for an AA dinucleotide sequences. The occurrence of each AA and the 3' adjacent 19 nucleotides are recorded as potential siRNA target sites. Tuschl et al. don't recommend designing siRNA to the 5' and 3' untranslated regions (UTRs) and regions near the start codon (within 75bases) as these may be richer in regulatory protein binding sites. UTR-binding proteins and/or translation initiation complexes may interfere with binding of the siRNA endonuclease complex.
2. The potential target sites are compared to the appropriate genome database (human, mouse, rat, etc.) to eliminate target sequences with significant homology to other coding sequences.
3. Qualifying target sequences are selected for synthesis. Several target sequences along the length of the gene are selected for evaluation.
The oligonucleotides used for siRNAs of PKP3, CDCA1, CDCA8, DLX5, URLC11 or NPTX1 are shown below. Each oligonucleotide is a combination of a sense nucleotide sequence and an antisense nucleotide sequence of the target sequence. The nucleotide sequences of the hairpin loop structure and target sequence of siRNAs are shown in SEQ ID NO:43 to SEQ ID NO:57 and SEQ ID NO:58 to SEQ ID NO:72, respectively (endonuclease recognition cites are eliminated from each hairpin loop structure sequence). Also, the nucleotide sequences of the hairpin loop structure and target sequence of control siRNAs (psiH 1BX-EGFP, psiH1BX-LUC or psiH1BX-SCR (Scramble)) are shown in SEQ ID NO:98 to SEQ ID NO:100 and SEQ ID NO:101 to SEQ ID NO:103, respectively.
Insert sequence of siRNA expression vectors for PKP3;
siRNA2:
   5'-TCCCCCTGTGGCAGTACAACAAGTTCAAGAGACTTGTTGTACTGCCACAGG-3'(SEQ ID NO: 13) and
   5'-AAAACCTGTGGCAGTACAACAAGTCTCTTGAACTTGTTGTACTGCCACAGG-3'(SEQ ID NO: 14)
Insert sequence of siRNA expression vectors for CDCA1;
siRNA1: 
5'-TCCCTGCCAGACAAGAAGTGGTGTTCAAGAGACACCACTTCTTGTCTGGCA-
3'(SEQ ID NO: 15)
 and 
5'-AAAATGCCAGACAAGAAGTGGTGTCTCTTGAACACCACTTCTTGTCTGGCA-
3'(SEQ ID NO: 16)
siRNA2: 
5'-TCCCGATGCTGCTGAAAGGGAGATTCAAGAGATCTCCCTTTCAGCAGCATC-
3'(SEQ ID NO: 17)
 and 
5'-AAAAGATGCTGCTGAAAGGGAGATCTCTTGAATCTCCCTTTCAGCAGCATC-
3'(SEQ ID NO: 18)
Insert sequence of siRNA expression vectors for CDCA8;
siRNA1: 
5'-TCCCCAGCAGAAGCTATTCAGACTTCAAGAGAGTCTGAATAGCTTCTGCTG-
3'(SEQ ID NO: 19)
 and 
5'-AAAACAGCAGAAGCTATTCAGACTCTCTTGAAGTCTGAATAGCTTCTGCTG-
3'(SEQ ID NO: 20)
siRNA2: 
5'-TCCCGGTGTCCTCCATCCAAGAATTCAAGAGATTCTTGGATGGAGGACACC-
3'(SEQ ID NO: 21)
 and 
5'-AAAAGGTGTCCTCCATCCAAGAATCTCTTGAATTCTTGGATGGAGGACACC-
3'(SEQ ID NO: 22)
siRNA3 : 
5'-TCCCGCCGTGCTAACACTGTTACTTCAAGAGAGTAACAGTGTTAGCACGGC-
3'(SEQ ID NO: 23)
 and 
5'-AAAAGCCGTGCTAACACTGTTACTCTCTTGAAGTAACAGTGTTAGCACGGC-
3'(SEQ ID NO: 24)
siRNA4: 
5'-TCCCGAAGCTCTCCAACCGTCTCTTCAAGAGAGAGACGGTTGGAGAGCTTC-
3'(SEQ ID NO: 25)
 and 
5'-AAAAGAAGCTCTCCAACCGTCTCTCTCTTGAAGAGACGGTTGGAGAGCTTC-
3'(SEQ ID NO: 26)
Insert sequence of siRNA expression vectors for DLX5;
siRNA2: 
5'-TCCCGACTCAGTACCTCGCCTTGTTCAAGAGACAAGGCGAGGTACTGAGTC-
3'(SEQ ID NO: 27)
 and 
5'-AAAAGACTCAGTACCTCGCCTTGTCTCTTGAACAAGGCGAGGTACTGAGTC-
3'(SEQ ID NO: 28)
siRNA6: 
5'- TCCCGGTTTCAGAAGACTCAGTATTCAAGAGATACTGAGTCTTCTGAAACC -
3'(SEQ ID NO: 29)
 and 
5'- AAAAGGTTTCAGAAGACTCAGTATCTCTTGAATACTGAGTCTTCTGAAACC -
3'(SEQ ID NO: 30)
siRNA7: 
5'- TCCCGTGCAGCCAGCTCAATCAATTCAAGAGATTGATTGAGCTGGCTGCAC -
3'(SEQ ID NO: 31)
 and 
5'- AAAAGTGCAGCCAGCTCAATCAATCTCTTGAATTGATTGAGCTGGCTGCAC -
3'(SEQ ID NO: 32)
Insert sequence of siRNA for expression vectors URLC11;
siRNA1: 
5'-TCCCGAGAATTCATTACTACAGCTTCAAGAGAGCTGTAGTAATGAATTCTC-
3'(SEQ ID NO: 33)
 and 
5'-AAAAGAGAATTCATTACTACAGCTCTCTTGAAGCTGTAGTAATGAATTCTC-
3'(SEQ ID NO: 34)
siRNA3: 
5'-TCCCGGATATTCCTGCTGTTCCATTCAAGAGATGGAACAGCAGGAATATCC-
3'(SEQ ID NO: 35)
 and 
5'-AAAAGGATATTCCTGCTGTTCCATCTCTTGAATGGAACAGCAGGAATATCC-
3'(SEQ ID NO: 36)
siRNA4: 
5'-TCCCGATATTCAGGAGCAGCATGTTCAAGAGACATGCTGCTCCTGAATATC-
3'(SEQ ID NO: 37)
 and 
5'-AAAAGATATTCAGGAGCAGCATGTCTCTTGAACATGCTGCTCCTGAATATC-
3'(SEQ ID NO: 38)
Insert sequence of siRNA for expression vectors NPTX1;
siRNA1: 
5'-TCCCGGAGACCATCCTGAGCCAGTTCAAGAGACTGGCTCAGGATGGTCTCC-
3'(SEQ ID NO: 39)
 and 
5'-AAAAGGAGACCATCCTGAGCCAGTCTCTTGAACTGGCTCAGGATGGTCTCC-
3'(SEQ ID NO: 40)
siRNA2: 
5'-TCCCGTGGACCTTCGAGGCCTGTTTCAAGAGAACAGGCCTCGAAGGTCCAC-
3'(SEQ ID NO: 41)
 and 
5'-AAAAGTGGACCTTCGAGGCCTGTTCTCTTGAAACAGGCCTCGAAGGTCCAC-
3'(SEQ ID NO: 42)
Insert sequence of siRNA expression vectors for control
psiH1BX-EGFP (EGFPsi)
   5'- CACCGAAGCAGCACGACTTCTTCTTCAAGAGAGAAGAAGTCGTGCTGCTTC-3' (SEQ ID NO; 92) and
   5'-AAAAGAAGCAGCACGACTTCTTCTCTCTTGAAGAAGAAGTCGTGCTGCTTC-3' (SEQ ID NO; 93)
psiHlBX-LUC (LUCsi)
   TCCCCGTACGCGGAATACTTCGATTCAAGAGATCGAAGTATTCCGCGTACG (SEQ ID NO; 94) and
   AAAACGTACGCGGAATACTTCGATCTCTTGAATCGAAGTATTCCGCGTACG (SEQ ID NO; 95)
psiHIBX-SCR (SCRsi)
   TCCCGCGCGCTTTGTAGGATTCGTTCAAGAGACGAATCCTACAAAGCGCGC (SEQ ID NO; 96) and
   AAAAGCGCGCTTTGTAGGATTCGTCTCTTGAACGAATCCTACAAAGCGCGC (SEQ ID NO; 97)
Sequence ID NO of each sequences are listed in Table 2

| Gene | siRNA | insert seq SEQ ID NO. | insert seq SEQ ID NO. | hairpin siRNA | target seq. | position |
|---|---|---|---|---|---|---|
| PKP3 | Si2 | 13 | 14 | 43 | 58 | 2393-2411 |
| | Si1 | 15 | 16 | 44 | 59 | 1099-1117 |
| CDCA1 | Si2 | 17 | 18 | 45 | 60 | 1526-1544 |
| | Si1 | 19 | 20 | 46 | 61 | 412-430 |
| | Si2 | 21 | 22 | 47 | 62 | 544-562 |
| | Si3 | 23 | 24 | 48 | 63 | 607-625 |
| CDCA8 | Si4 | 25 | 26 | 49 | 64 | 902-920 |
| | Si2 | 27 | 28 | 50 | 65 | 668-686 |
| | Si6 | 29 | 30 | 51 | 66 | 658-676 |
| DLX5 | Si7 | 31 | 32 | 52 | 67 | 979-997 |
| | Si1 | 33 | 34 | 53 | 68 | 331-349 |
| | Si3 | 35 | 36 | 54 | 69 | 1464-1482 |
| URLC11 | Si4 | 37 | 38 | 55 | 70 | 1653-1671 |
| | Si1 | 39 | 40 | 56 | 71 | 339-357 |
| NPTX1 | Si2 | 41 | 42 | 57 | 72 | 1398-1416 |
| control | EGFPsi | 92 | 93 | 98 | 101 | |
| control | LUCsi | 94 | 95 | 99 | 102 | |
| control | SCRsi | 96 | 97 | 100 | 103 | |

### RNA interference assay

A vector-based RNAi system, psiH1BX3.0, which directs the synthesis of small interfering RNAs (siRNAs) in mammalian cells was used for suppressing the expression of each of the endogenous genes in NSCLC cells. The vectors were transfected into NSCLC cell lines using Lipofectamine 2000 reagent (Invitrogene, Carlsbad, CA, USA), which resulted in transfection with more than 60-90% transfection efficiency. Cells were cultured for 5-9 days in the presence of an appropriate concentration of geneticin (G418). The cell numbers or cell viability was determined upon Giemsa staining and/or MTT assay in triplicate.

### Immunocytochemical analysis

To prepare c-myc-His tagged proteins, pcDNA3.1 (+)/c-myc-His, a plasmid containing genes encoding the c-myc-His epitope sequence (LDEESILKQE-HHHHHH) at the C-terminus of each protein were constructed and transfected into COS-7 cells. The transiently transfected COS-7 cells replanted on chamber slides were fixed with PBS containing 4% paraformaldehyde, then rendered permeable with PBS containing 0.1 % Triton X-100 for 3 min at 4°C. The cells were covered with blocking solution (2% BSA in PBS) for 30 min at room temperature to block nonspecific antibody-binding sites. Then the cells were incubated with mouse anti-c-myc antibody (diluted 1: 800 in blocking solution). The antibody was stained with goat antimouse secondary antibody conjugated with FITC to observe them under ECLIPSE E800 microscope (Nikon). Specifically for PKP3, the cells were stained with mouse anti-c-myc-rhodamine antibody (diluted 1: 800 in blocking solution), or anti-PKP3 antibody conjugated with FITC to observe them under ECLIPSE E800 microscope (Nikon).

### 3-(4.5-dimethylthiazol-2-yl)-2,5-diphenyltetrazolum bromide (MTT) assay

Cells were transfected with psiHIBX3.0-siRNAs or control plamids and maintained in the culture media supplemented with optimum concentration of geneticin. Six to twelve days after transfection, the medium was replaced with fresh medium containing 500 µg/ml of MTT (3-(4,5-dimethylthiazol-2-yl)-2,5-diphenyl tetrazolium bromide) (Sigma) and the plates were incubated for four hours at 37°C. Subsequently, the cells were lysed by the addition of 1 ml of 0.01 N HCI/10%SDS and. absorbance of lysates was measured with an ELISA plate reader at a test wavelength of 570 nm (reference, 630 nm). The cell viability was represented by the absorbance compared to that of control cells.

### Matrigel invasion assay

COS-7 cells transiently transfected with plasmids expressing *PKP3* or mock plasmids were grown to near confluence in DMEM containing 10% fetal bovine serum. The cells were harvested by trypsinization and subsequently washed in DMEM without addition of serum or proteinase inhibitor. The cells were suspended in DMEM at 1 x 10⁵/ml. Before preparing the cell suspension, the dried layer of Matrigel matrix (Becton Dickinson Labware, Bedford, MA, USA) was rehydrated with DMEM for 2 hours at room temperature. DMEM (0.75 ml) containing 10% fetal bovine serum was added to each lower chamber of 24-well Matrigel invasion chambers, and 0.5 ml (5 x 10⁴ cells) of cell suspension was added to each insert of the upper chamber. The plates of inserts were incubated for 22 hours at 37 °C. After incubation the chambers were processed and the cells invading through the Matrigel-coated inserts were fixed and stained by Giemsa as directed by the supplier (Becton Dickinson).

### [Example 2] Results

Screening of up-regulated genes by cDNA microarrays containing 23,040 genes (Kikuchi, T., Daigo, Y., Katagiri, T., Tsunoda, T., Okada, K., Kakiuchi, S., Zembutsu, H., Furukawa, Y., Kawamura, M., Kobayashi, K., Imai, K., and Nakamura, Y. Expression profiles of non-small cell lung cancers on cDNA microarrays: identification of genes for prediction of lymph-node metastasis and sensitivity to anti-cancer drugs, Oncogene. 22: 2192-205, 2003.) was performed previously. By pursuing such a strategy, abundant expression of six genes in the great majority of clinical NSCLC and cell lines were identified. In the present invention, six genes: plakophilin 3 (PKP3), cell division cycle associated 1 (CDCA1), cell division cycle associated 8 (*CDCA8*), distal-less homeo box 5 (*DLX5*), *URLC11* and neuronal pentraxin 1 (*NPTX1*) are revealed to be potential molecular targets for treatment of lung cancers.

### (1) PKP3: (GENBANK ACCESSIONNO.NM_007183)

### Northern-blot

Northern-blot analysis using human *PKP3* cDNA as a probe detected a transcript of 3-kb in heart, placenta, pancreas, prostate, small intestine, stomach, thyroid, and trachea (Figure 1a).

### RNAi

To assess whether PKP3 is essential for growth or survival of lung-cancer cells, we designed and constructed plasmids to express siRNA against *PKP3* (si2), and three different control plasmids (siRNAs for EGFP, Luciferase (LUC), or Scramble (SCR)), and transfected them into A549 and LC319 cells to suppress expression of endogenous *PKP3.* The amount of *PKP3* transcript in the cells transfected with si2 was significantly decreased in comparison with cells transfected with any of the three control siRNAs(Figurelb, I c); transfection of si2 also resulted in significant decreases in cell viability and colony numbers measured by MTT(Figureld, 1e) and colony-formation assays(data not shown).

### Immunocytochemical analysis

To determine the subcellular localization of the protein encoded by this gene, COS-7 cells were transfected with the c-myc-His tagged PKP3 expression plasmid. We detected the PKP3/c-myc-His protein mainly in perinucleus and cytoplasmic membrane by immnocytochemical analysis(Figure1f).

### Matrigel invasion assay

Matrigel invasion assays were performed with COS-7-PKP3 cells. Invasion of COS-*7-PKP3* cells through Matrigel was significantly promoted, compared to the control cells transfected with mock plasmids (Figure1g).

### (2) CDCA1:(GENBANK ACCESSION NO.NM_145697)

### Northern-blot

Northern-blot analysis using human *CDCA1* cDNA as a probe detected a transcript of 2.4-kb in testis (Figure2a).

### RNAi

To assess whether CDCA1 is essential for growth or survival of lung-cancer cells, we designed and constructed plasmids to express siRNA against *CDCA1* (si1, si2), and three different control plasmids (siRNAs for EGFP, LUC, or SCR), and transfected them into LC319 cells to suppress expression of endogenous *CDCA1.* The amount of *CDCA1* transcript in the cells transfected with si1 and si2 were significantly decreased in comparison with cells transfected with any of the three control siRNAs(Figure2b); transfection of si1 and si2 also resulted in significant decreases in cell viability and colony numbers measured by MTT(Figure2c) and colony-formation assays (data not shown).

### Immunocytochemical analysis

To determine the subcellular localization of the protein encoded by this gene, COS-7 cells were transfected with the c-myc-His tagged CDCA1 expression plasmid. We detected the CDCA1/c-myc-His protein mainly in nucleus and cytoplasm by immunocytochemical analysis (data not shown).

### (3) CDCA8: (GENBANK ACCESSION NO.NM_018101)

### Northern-blot

Northern-blot analysis using human *CDCA8* cDNA as a probe detected a transcript of 2.4-kb in testis (Figure3a).

### RNAi

To assess whether CDCA8 is essential for growth or survival of lung-cancer cells, we designed and constructed plasmids to express siRNA against *CDCA8* (si1, si2, si3, si4), and three different control plasmids (siRNAs for EGFP, LUC, or SCR), and transfected them into LC319 (data not shown) and A549 cells to suppress expression of endogenous *CDCA8.* The amount of *CDCA8* transcript in the cells transfected with si1, si2, si3, and si4 were significantly decreased in comparison with cells transfected with any of the three control siRNAs(Figure3b); transfection of si1, si2, si3, and si4 also resulted in significant decreases in colony numbers measured by colony-formation assays (data not shown).

### Immunocytochemical analysis

To determine the subcellular localization of the protein encoded by this gene, COS-7 cells were transfected with the c-myc-His tagged CDCA8 expression plasmid. We detected the CDCA8/c-myc-His protein mainly in nucleus and cytoplasm by immunocytochemical analysis (data not shown).

### (4) DLX5: (GEANK ACCESSION NO. BC006226)

### Northern-blot

Northern-blot analysis using human *DLX5* cDNA as a probe detected a transcript of 1.5-kb in placenta (Figure4a).

### RNAi

To assess whether *DLY5* is essential for growth or survival of lung-cancer cells, we designed and constructed plasmids to express siRNA against *DLY5* (si2, si6, si7), and three different control plasmids (siRNA for EGFP, LUC, or SCR), and transfected them into LC319 cells to suppress expression of endogenous *DLY5.* The amount of *DLX5* transcript in the cells transfected with si2, si6 and si7 were significantly decreased in comparison with cells transfected with the control siRNA(Figure4b); transfection of si2, si6 and si7 also resulted in significant decreases in cell viability and colony numbers measured by MTT(Figure4c) and colony-formation assays(data not shown).

### (5) URLC11: (GENBANK ACCESSION NO.AB105188)

### RNAi

To assess whether *URLC11* is essential for growth or survival of lung-cancer cells, we designed and constructed plasmids to express siRNA against URLC11 (si1, si3, si4) and three different control plasmids (siRNAs for EGFP, LUC, or SCR), and transfected them into A549 cells to suppress expression of endogenous *URLC11*. The amount of *URLC11* transcript in the cells transfected with si1, si3, and si4 were significantly decreased in comparison with cells transfected with any of the three control siRNAs (Figure5a); transfection of si1, si3, and si4 also resulted in significant decreases in cell viability and colony numbers measured by MTT(Figure5b) and colony-formation assays(data not shown).

### (6) NPTX1:(GENBANK ACCESSION NO.NM_002522)

### RNAi

To assess whether *NPTX1* is essential for growth or survival of lung-cancer cells, we designed and constructed plasmids to express siRNA against *NPTX1* (si1, si2), and three different control plasmids (siRNAs for EGFP, LUC, or SCR), and transfected them into A549 and LC176 cells to suppress expression of endogenous *NPTX1.* The amount of *NPTX1* transcript in the cells transfected with si1 and si2 were significantly decreased in comparison with cells transfected with any of the three control siRNAs(Figure6a, 6b); transfection of si1 and si2 also resulted in significant decreases in cell viability and colony numbers measured by MTT(Figure6c, 6d) and colony-formation assays(data not shown).

### Industrial Applicability

The present inventors have shown that the cell growth is suppressed by inhibiting expression of the PKP3, CDCA1, CDCA8, DLX5, URLC11or NPTX1 gene. In particular, small interfering RNA (siRNA) that specifically target of these genes have been effective in suppressing cell growth. Thus, this novel siRNAs are useful target for the development of anti-cancer pharmaceuticals. For example, agents that block the expression of PKP3, CDCA1, CDCA8, DLX5, URLC11 or NPTX1 or prevent its activity may find therapeutic utility as anti-cancer agents, particularly anti-cancer agents for the treatment of lung cancer, such as NSCLC.

While the invention has been described in detail and with reference to specific embodiments thereof, it will be apparent to one skilled in the art that various changes and modifications can be made therein without departing from the spirit and scope of the invention.
Furthermore; the present invention relates to the following items:
1. A method of treating or preventing lung cancer in a subject comprising administering to said subject a composition comprising a small interfering RNA (siRNA) that inhibits expression of PKP3, CDCA1, CDCA8, DLX5, URLC11 or NPTX1.
2. The method of item 1, wherein said siRNA comprises a sense nucleic acid sequence and an anti-sense nucleic acid sequence that specifically hybridizes to a sequence from PKP3, CDCA1, CDCA8, DLX5, URLC11 or NPTX1.
3. The method of item 1, wherein the lung cancer is an non-small cell lung cancer (NSCLC).
4. The method of item 2, wherein said siRNA comprises a ribonucleotide sequence corresponding to a sequence selected from the group consisting of SEQ ID NOs: 58, 59, 60, 61, 62, 63, 64, 65, 66, 67, 68, 69, 70, 71 or 72 as the target sequence.
5. The method of item 4, wherein said siRNA has the general formula 5'-[A]-[B]-[A']-3', wherein
   [A] is a ribonucleotide sequence corresponding to a sequence selected from the group consisting of nucleotides of SEQ ID NOs: 58, 59, 60, 61, 62, 63, 64, 65, 66, 67, 68, 69, 70, 71 or 72.
   [B] is a ribonucleotide sequence consisting of about 3 to about 23 nucleotides, and
   [A'] is a ribonucleotide sequence consisting of the complementary sequence of [A].
6. The method of item 1, wherein said composition comprises a transfection-enhancing agent.
7. A double-stranded molecule comprising a sense strand and an antisense strand, wherein the sense strand comprises a ribonucleotide sequence corresponding to a target sequence selected from the group consisting of SEQ ID NOs: 58, 59, 60, 61, 62, 63, 64, 65, 66, 67, 68, 69, 70, 71 or 72, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the PKP3, CDCA1, CDCA8, DLX5, URLC11 or NPTX1 gene, inhibits expression of said gene.
8. The double-stranded molecule of item 7, wherein said target sequence comprises at least about 10 contiguous nucleotides from the nucleotide sequences selected from the group of SEQ ID NOs: 1, 3, 5, 7, 9 or 11.
9. The double-stranded molecule of item 8, wherein said target sequence comprises from about 19 to about 25 contiguous nucleotides from the nucleotide sequences selected from the group of SEQ ID NOs: 1, 3, 5, 7, 9 or11.
10. The double-stranded molecule of item 9, wherein said double-stranded molecule is a single ribonucleotide transcript comprising the sense strand and the antisense strand linked via a single-stranded ribonucleotide sequence.
11. The double-stranded molecule of item 8, wherein the double-stranded molecule is an oligonucleotide of less than about 100 nucleotides in length.
12. The double-stranded molecule of item 11, wherein the double-stranded molecule is an oligonucleotide of less than about 75 nucleotides in length.
13. The double-stranded molecule of item 12, wherein the double-stranded molecule is an oligonucleotide of less than about 50 nucleotides in length.
14. The double-stranded molecule of item 13, wherein the double-stranded molecule is an oligonucleotide of less than about 25 nucleotides in length.
15. The double-stranded polynucleotide of item 14, wherein the double stranded molecule is an oligonucleotide of between about 19 and about 25 nucleotides in length.
16. A vector encoding the double-stranded molecule of item 7.
17. The vector of item 16, wherein the vector encodes a transcript having a secondary structure and comprises the sense strand and the antisense strand.
18. The vector of item 17, wherein the transcript further comprises a single-stranded ribonucleotide sequence linking said sense strand and said antisense strand.
19. A vector comprising a polynucleotide comprising a combination of a sense strand nucleic acid and an antisense strand nucleic acid, wherein said sense strand nucleic acid comprises nucleotide sequence of SEQ ID NOs: 58, 59, 60, 61, 62, 63, 64, 65, 66, 67, 68, 69, 70, 71 or 72, and said antisense strand nucleic acid consists of a sequence complementary to the sense strand.
20. The vector of item 19, wherein said polynucleotide has the general formula 5'-[A]-[B]-[A']-3'
   wherein [A] is a nucleotide sequence of SEQ ID NOs: 58, 59, 60, 61, 62, 63, 64, 65, 66, 67, 68, 69, 70, 71 or 72; [B] is a nucleotide sequence consisting of about 3 to about 23 nucleotides; and [A'] is a nucleotide sequence complementary to [A].
21.A pharmaceutical composition for treating or preventing lung cancer comprising a pharmaceutically effective amount of a small interfering RNA (siRNA) that inhibits expression of PKP3, CDCA1, CDCA8, DLX5, URLC11 or NPTX1 as an active ingredient, and a pharmaceutically acceptable carrier.
22. The pharmaceutical composition of item 21, wherein the siRNA comprises a nucleotide sequence selected from the group consisting of SEQ ID NOs: 58, 59, 60, 61, 62, 63, 64, 65, 66, 67, 68, 69, 70, 71 or 72 as the target sequence.
23. The composition of item 22, wherein the siRNA has the general formula 5'-[A]-[B]-[A']-3'
   wherein [A] is a ribonucleotide sequence corresponding to a nucleotide sequence of SEQ ID NOs: 58, 59, 60, 61, 62, 63, 64, 65, 66, 67, 68, 69, 70, 71 or 72; [B] is a ribonucleotide sequence consisting of about 3 to about 23 nucleotides; and [A'] is a ribonucleotide sequence complementary to [A].

## Claims

1. A double-stranded molecule comprising a sense strand and an antisense strand, wherein the sense strand comprises a ribonucleotide sequence corresponding to a target sequence selected from the group consisting of SEQ ID NOs: 59, 60, 58, 61, 62, 63, 64, 65, 66, 67, 68, 69, 70, 71 or 72, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the CDCA1, PKP3, CDCA8, DLX5, URLC11 or NPTX1 gene, inhibits expression of said gene.

2. The double-stranded molecule of claim 1, wherein said double-stranded molecule is a single ribonucleotide transcript comprising the sense strand and the antisense strand linked via a single-stranded ribonucleotide sequence.

3. The double-stranded polynucleotide of claim 1, wherein the double stranded molecule is an oligonucleotide of between about 19 and about 25 nucleotides in length.

4. A vector encoding the double-stranded molecule of any one of claims 1 to 3.

5. The vector of claim 4, wherein the vector encodes a transcript having a secondary structure and comprises the sense strand and the antisense strand.

6. The vector of claim 5, wherein the transcript further comprises a single-stranded ribonucleotide sequence linking said sense strand and said antisense strand.

7. A vector comprising a polynucleotide comprising a combination of a sense strand nucleic acid and an antisense strand nucleic acid, wherein said sense strand nucleic acid comprises nucleotide sequence of SEQ ID NOs: 59, 60, 58, 61, 62, 63, 64, 65, 66, 67, 68, 69, 70, 71 or 72, and said antisense strand nucleic acid consists of a sequence complementary to the sense strand.

8. The vector of claim 7, wherein said polynucleotide has the general formula
5'-[A]-[B]-[A']-3'
wherein [A] is a nucleotide sequence of SEQ ID NOs: 59, 60, 58, 61, 62, 63, 64, 65, 66, 67, 68, 69, 70, 71 or 72; [B] is a nucleotide sequence consisting of about 3 to about 23 nucleotides; and [A'] is a nucleotide sequence complementary to [A].

9. Use of a small interfering RNA (siRNA) that inhibits expression of CDCA1, PKP3, CDCA8, DLX5, URLC11 or NPTX1 or a vector encoding the small interfering RNA for the preparation of a pharmaceutical composition for treating or preventing lung cancer.

10. Use of claim 9, wherein said siRNA comprises a sense nucleic acid sequence and an anti-sense nucleic acid sequence that specifically hybridizes to a sequence from CDCA1, PKP3, CDCA8, DLX5, URLC11 or NPTX1.

11. Use of claim 9, wherein the lung cancer is an non-small cell lung cancer (NSCLC).

12. Use of claim 9, wherein said composition comprises a transfection-enhancing agent.

13. A pharmaceutical composition for use in treating or preventing lung cancer comprising a pharmaceutically effective amount of a small interfering RNA (siRNA) that inhibits expression of CDCA1, PKP3, CDCA8, DLX5, URLC11 or NPTX1, or a vector encoding the small interfering RNA as an active ingredient, and a pharmaceutically acceptable carrier.

14. The use of claim 2 or the pharmaceutical composition of claim 13, wherein the siRNA comprises a nucleotide sequence selected from the group consisting of SEQ ID NOs: 59, 60, 58, 61, 62, 63, 64, 65, 66, 67, 68, 69, 70, 71 or 72 as the target sequence.

15. Use or the composition of claim 14, wherein the siRNA has the general formula
5'-[A]-[B]-[A']-3'
wherein [A] is a ribonucleotide sequence corresponding to a nucleotide sequence of SEQ ID NOs: 59, 60, 58, 61, 62, 63, 64, 65, 66, 67, 68, 69, 70, 71 or 72; [B] is a ribonucleotide sequence consisting of absout 3 to about 23 nucleotides; and [A'] is a ribonucleotide sequence complementary to [A].
